# EUROPEAN PATENT APPLICATION

(11) **EP 2 017 282 A1**
(43) Date of publication of application: **21.01.2009**
(21) Application number: 07714923.5
(22) Date of filing: 26.02.2007
(51) Int. Cl.: C07H 21/00

(54) **METHOD OF CAPPING OLIGONUCLEIC ACID**

(30) Priority: 27.02.2006 JP 2006050389
(71) Applicant: Nippon Shinyaku Co., Ltd., Kyoto-shi, Kyoto 601-8550 (JP)
(72) Inventor: ENYA, Yukiko, Funabashi-shi, Chiba 274-0826 (JP)
(74) Representative: Vossius & Partner
(86) International application number: PCT/JP2007/053491
(87) International publication number: WO 2007/097446

(57) **Abstract**

The main object of the present invention is to provide a method for efficiently acylating the 5'-hydroxyl group of a ribose in a so-called capping step.
A method for producing an oligonucleic acid derivative represented by the following general formula (12), **characterized by** using a phenoxyacetic acid derivative anhydride represented by the following general formula (11a) as an acylating agent and a pyridine derivative represented by the following general formula (11b) or (11c) as the acylation reaction activator, in a capping step for protecting the 5'-hydroxyl group of a ribose of an oligonucleic acid derivative represented by the following general formula (2) with an acyl group.

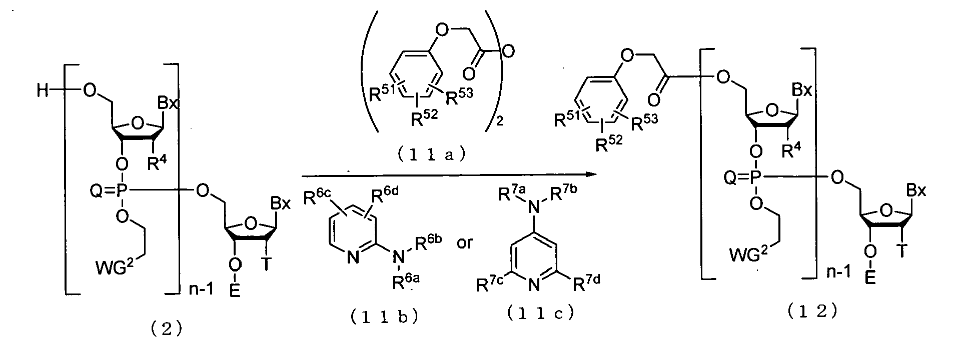

## Description

### FIELD OF THE INVENTION

The present invention relates to a capping step in a method for the solid-phase synthesis of an oligonucleic acid.

### BACKGROUND ART

As a method for producing an oligonucleic acid, a solid-phase synthesis method is known (Non-patent document 1). The solid-phase synthesis method is a method for producing an oligonucleic acid having a desired chain length by coupling an oligonucleic acid derivative immobilized on a solid support with a phosphoramidite compound. However, the phosphoramidite compound does not always completely react with all the oligonucleic acid derivatives immobilized on the solid support. Therefore, in order to produce a high purity oligonucleic acid by the solid-phase synthesis method, it is necessary to carry out a step in which the 5'-hydroxyl groups of the unreacted oligonucleic acid derivatives immobilized on the solid support are protected so as not to be involved in the above-mentioned coupling reaction, i.e., a so-called capping step.
In the capping step, as an acylating agent for protecting the 5'-hydroxyl group of the oligonucleic acid derivative, an acid anhydride (for example, acetic anhydride or phenoxyacetic anhydride) is generally used, and as the acylation reaction activator, for example, 4-dimethylaminopyridine (hereinafter referred to as "4-DMAP") and N-methylimidazole (hereinafter referred to as "NMI") are generally used.
However, it has been reported that when 4-DMAP is used as the acylation reaction activator, the nucleobase guanine reacts with 4-DMAP and is converted into 2,6-diaminopurine (hereinafter referred to as "2,6-DAP") (Non-patent document 2).
Non-patent document 1: Agrawal et al., Methods in Molecular Biology: Protocols for Oligonucleic acids and Analogs; Humana Press: Totowa, Vol. 20, 63 (1993)
Non-patent document 2: J. Scott et al., Nucleic Acids Research, Vol. 17, No. 20, 8333 (1987)

### DISCLOSURE OF THE INVENTION

### PROBLEM TO BE SOLVED BY THE INVENTION

NMI is currently the most generally used acylation reaction activator. However, when the present inventors performed the capping step for an oligonucleic acid derivative represented by the following general formula (2) using phenoxyacetic anhydride and NMI, they found a problem as shown in the Test examples described later; that is, phenoxyacetyl was not introduced into the 5'-hydroxyl group with a satisfactory efficiency, and as a result, the purification procedure for obtaining a high purity oligonucleic acid became complicated.
The main object of the present invention is to provide a method for efficiently acylating the 5'-hydroxyl group of a ribose in the so-called capping step.

### MEANS TO SOLVE THE PROBLEM

As a result of extensive studies, the present inventors have found that an oligonucleic acid derivative represented by the following general formula (12) can be efficiently produced by using a phenoxyacetic acid derivative anhydride represented by the following general formula (11a) as an acylating agent and a pyridine derivative represented by the following general formula (11b or (11c) as the acylation reaction activator in a capping step for protecting the 5'-hydroxyl group of a ribose of an oligonucleic acid derivative represented by the following general formula (2) with an acyl group, and thus the present invention has been completed. 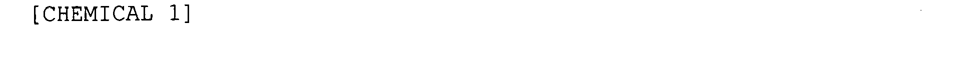 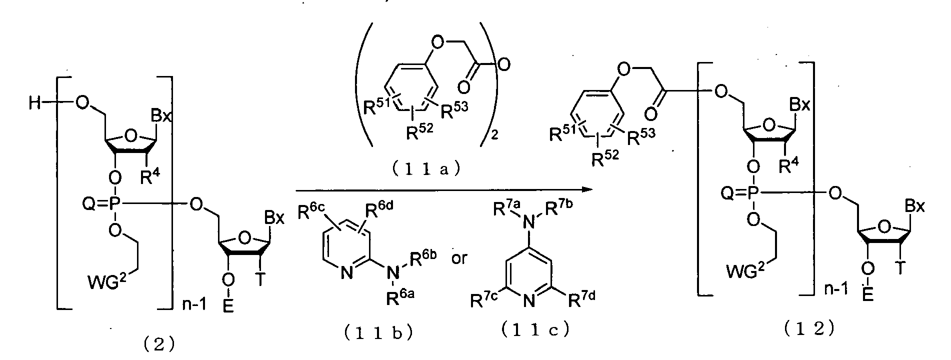 In the general formulae (2), (11a), (11b), (11c) and (12), each B_{X} independently represents a nucleobase which may have protecting groups or a modified form thereof. n represents an integer in the range of 1 to 200. n is preferably an integer in the range of 10 to 100, and more preferably an integer in the range of 15 to 50. Each Q independently represents O or S. R⁵¹, R⁵² and R⁵³ are the same or different and each represents H, alkyl or halogen. R^{6a}, R^{6b}, R^{7a}, R^{7b}, R^{7c} and R^{7d} are the same or different and each represents alkyl. R^{6c} and R^{6d} are the same or different and each represents H or alkyl. Each WG² represents an electron-withdrawing group. Each R⁴ independently represents H, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy or a substituent represented by the following general formula (3). 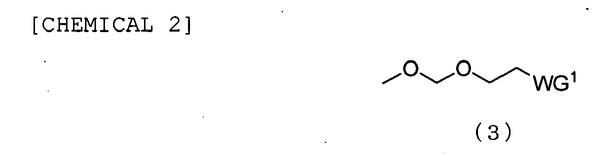 In the general formula (3), WG¹ represents an electron-withdrawing group. E represents acyl or a substituent represented by the following general formula (4). 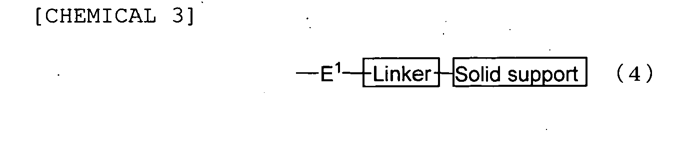 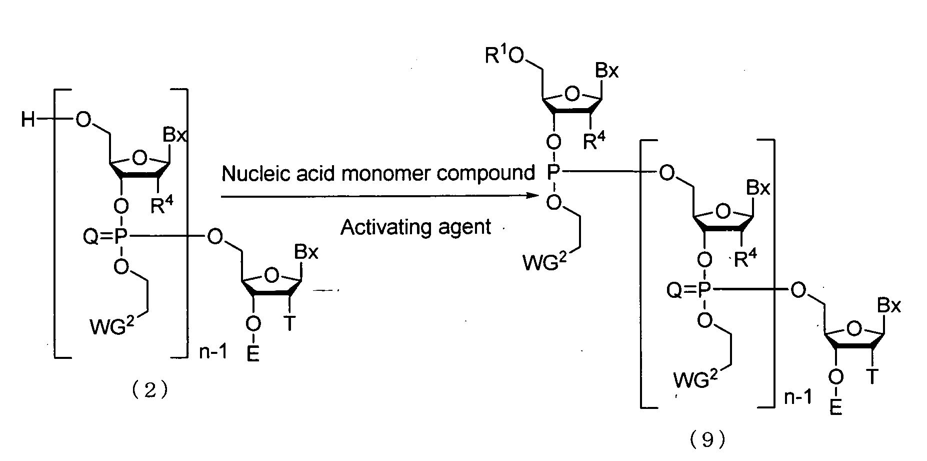 In the general formula (4), E¹ represents a single bond or a substituent represented by the following general formula (5). 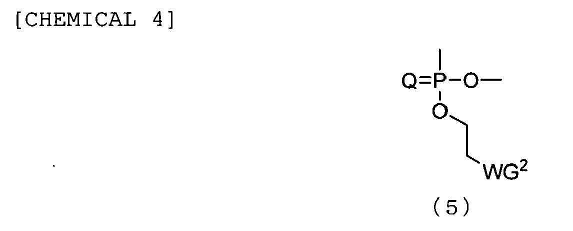 In the general formula (5), Q and WG² have the same meanings as above. T represents H, acyloxy, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy, a substituent represented by the general formula (3) or a substituent represented by the general formula (4), with the proviso that E or T is a substituent (4).
The "nucleobase" related to B_{X} is not particularly limited as long as it is a nucleobase to be used in the synthesis of a nucleic acid, and examples thereof may include pyrimidine bases such as cytosine, uracil and thymine, and purine bases such as adenine and guanine. The nucleobase related to B_{X} may be protected, and particularly in the case of a nucleobase having an amino group such as adenine, guanine and cytosine, the amino group thereof is preferably protected. The "protecting group of the amino group" is not particularly limited as long as it is a protecting group to be used as a protecting group of a nucleic acid, and specific examples may include benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. In particular, as the protecting group for the amino group of guanine, phenoxyacetyl, 4-tert-butylphenoxyacetyl and 4-isopropylphenoxyacetyl are preferred.
The "modified form" of B_{X} is a group in which a nucleobase has been substituted with an arbitrary substituent. Examples of the "substituent" may include halogen, acyl, alkyl, arylalkyl, alkoxy, alkoxyalkyl, hydroxyl, amino, monoalkylamino, dialkylamino, carboxy, cyano, and nitro. The modified form of B_{X} may be substituted by 1 to 3 of these substituents at arbitrary positions.
Examples of the "halogen" related to the modified form of B_{X} may include fluorine, chlorine, bromine and iodine.
Examples of the "acyl" related to the modified form of B_{X} may include straight or branched alkanoyl having 1 to 6 carbon atoms and aroyl having 7 to 13 carbon atoms. Specifically, the acyl may include, for example, formyl, acetyl, n-propionyl, isopropionyl, n-butyryl, isobutyryl, tert-butyryl, valeryl, hexanoyl, benzoyl, naphthoyl and levulinyl.
Examples of the "alkyl" related to the modified form of B_{X} may include straight or branched alkyl having 1 to 5 carbon atoms. Specifically, the alkyl may include, for example, methyl, ethyl, n-propyl, isopropyl, n-butyl, isobutyl, sec-butyl, tert-butyl, n-pentyl, isopentyl, neopentyl and tert-pentyl. The alkyl may be substituted, and examples of the "substituent" may include halogen, alkyl, alkoxy, cyano, and nitro. The alkyl may be substituted by 1 to 3 of these substituents at arbitrary positions.
Examples of the "alkyl" moiety of the "arylalkyl", "alkoxyalkyl", "monoalkylamino" and "dialkylamino" related to the modified form of B_{X} may include the same ones as those illustrated for the above-mentioned "alkyl".
Examples of the "alkoxy" related to the modified form of B_{X} may include straight or branched alkoxy having 1 to 4 carbon atoms. Specifically, the alkoxy may include, for example, methoxy, ethoxy, n-propoxy, isopropoxy, n-butoxy, isobutoxy, sec-butoxy and tert-butoxy. Among these, alkoxy groups having 1 to 3 carbon atoms are preferable, and methoxy is particularly preferable.
Examples of the "alkoxy" moiety of the "alkoxyalkyl" related to the modified form of B_{X} may include the same ones as those illustrated for the above-mentioned "alkoxy".
Examples of the "aryl" moiety of the "arylalkyl" related to the modified form of B_{X} may include aryl groups having 6 to 12 carbon atoms. Specifically, the aryl may include, for example, phenyl, 1-naphthyl, 2-naphthyl and biphenyl. The aryl may be substituted, and examples of the "substituent" may include halogen, alkyl, alkoxy, cyano and nitro. The aryl may be substituted by 1 to 3 of these substituents at arbitrary positions.
Examples of the "electron-withdrawing group" of WG¹ and WG² may include cyano, nitro, alkylsulfonyl, arylsulfonyl and halogen. Among them, cyano is preferred.
Examples of the "halogen" related to the electron-withdrawing group of WG¹ and WG² may include the same ones as those illustrated for the "halogen" related to the above-mentioned modified form of B_{X}.
Examples of the "alkyl" moiety of the "alkylsulfonyl" related to the WG¹ and WG² may include the same ones as those illustrated for the "alkyl" related to the above-mentioned modified form of B_{X}.
Examples of the "aryl" moiety of the "arylsulfonyl" related to the WG¹ and WG² may include the same ones as those illustrated for the "aryl" moiety of "arylalkyl" related to the above-mentioned modified form of B_{X}.
Examples of the "halogen", "alkoxy", "alkylamino" and "dialkylamino" related to R⁴ may include the same ones as those related to the above-mentioned modified form of B_{X}.
Examples of the "alkyl" moiety of the "alkoxyalkyloxy" or "alkylthio" related to R⁴ may include the same ones as those illustrated for the "alkyl" related to the above-mentioned modified form of B_{X}.
Examples of the "alkoxy" moiety of the "alkoxyalkyloxy" related to R⁴ may include the same ones as those illustrated for the "alkoxy" related to the above-mentioned modified form of B_{X}.
Examples of the "alkenyl" moiety of the "alkenyloxy", "alkenylthio", "alkenylamino" or "dialkenylamino" related to R⁴ may include straight or branched alkenyl having 2 to 6 carbon atoms. Specifically, the alkenyl may include, for example, vinyl, allyl, 1-propenyl, isopropenyl, 1-butenyl, 2-butenyl, 1-pentenyl and 1-hexenyl.
Examples of the "alkynyl" moiety of "alkynyloxy", "alkynylthio", "alkynylamino" or "dialkynylamino" related to R⁴ may include straight or branched alkynyl having 2 to 4 carbon atoms. Specifically, the alkynyl may include, for example, ethynyl, 2-propynyl and 1-butynyl.
Examples of the "alkyl" related to R⁵¹, R⁵², R⁵³, R^{6a}, R^{6b}, R^{6c}, R^{6d}, R^{7a}, R^{7b}, R^{7c} and R^{7d} may include the same ones as those illustrated for the "alkyl" related to the above-mentioned modified form of B_{X}.
Examples of the "halogen" related to R⁵¹, R⁵² and R⁵³ may include the same ones as those illustrated for the "halogen" related to the above-mentioned modified form of B_{X}.
Examples of the "acyl" related to the E may include the same ones illustrated for the "acyl" related to the above-mentioned modified form of B_{X}.
Examples of the "acyl" moiety of the "acyloxy" related to the T may include the same ones as those illustrated for the "acyl" related to the above-mentioned modified form of B_{X}.
Examples of the "halogen", "alkoxy", "alkylamino" or "dialkylamino" related to the T may include the same ones as those related to the above-mentioned modified form of B_{X}.
Examples of the "alkyl" moiety of "alkoxyalkyloxy" and "alkylthio" related to the T may include the same ones as those illustrated for the alkyl related to the above-mentioned modified form of B_{X}.
Examples of the "alkoxy" moiety of the "alkoxyalkyloxy" related to the T may include the same ones as those illustrated for the "alkoxy" related to the above-mentioned modified form of B_{X}.
Examples of the "alkenyl" moiety of the "alkenyloxy", "alkenylthio", "alkenylamino" and "dialkenylamino" related to the T may include the same ones as those illustrated for the "alkenyl" related to the above-mentioned R⁴.
Examples of the "alkynyl" moiety of "alkynyloxy", "alkynylthio", "alkynylamino" and "dialkynylamino" related to the T may include the same ones as those illustrated for the "alkynyl" related to the above-mentioned R⁴.
The "alkylamino", "alkenylamino" or "alkynylamino" related to the T, may be protected. The protecting group of the amino group is not particularly limited as long as it is a protecting group to be used as a protecting group for an amino group, and examples thereof may include trifluoroacetyl, benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. In particular, trifluoroacetyl is preferred.
The "acylating agent" for capping the 5'-hydroxyl group of a ribose can be exemplified by a phenoxyacetic acid derivative anhydride represented by the above-mentioned formula (11a), and examples thereof may include phenoxyacetic anhydride, 4-isopropylphenoxyacetic anhydride, 4-tert-butylphenoxyacetic anhydride and 4-chlorophenoxyacetic anhydride.
The above-mentioned "acylation reaction activator" can be exemplified by pyridine derivatives (11b) and (11c), and examples thereof may include 2-dimethylaminopyridine (2-DMAP), 2,6-di-tert-butyl-4-dimethylaminopyridine and 2,6-dimethyl-4-dimethylaminopyridine.
Further, as the present invention, a method for producing an oligonucleic acid represented by the following general formula (A) (hereinafter referred to as an "oligonucleic acid (A)") including the step of producing an oligonucleic acid derivative represented by the following general formula (12) by using a phenoxyacetic acid derivative anhydride represented by the following general formula (11a) as an acylating agent and a pyridine derivative represented by the following general formula (11b) or (11c) as the acylation reaction activator in a capping step for protecting the 5'-hydroxyl group of a ribose of an oligonucleic acid derivative represented by the following general formula (2) with an acyl group can be exemplified. 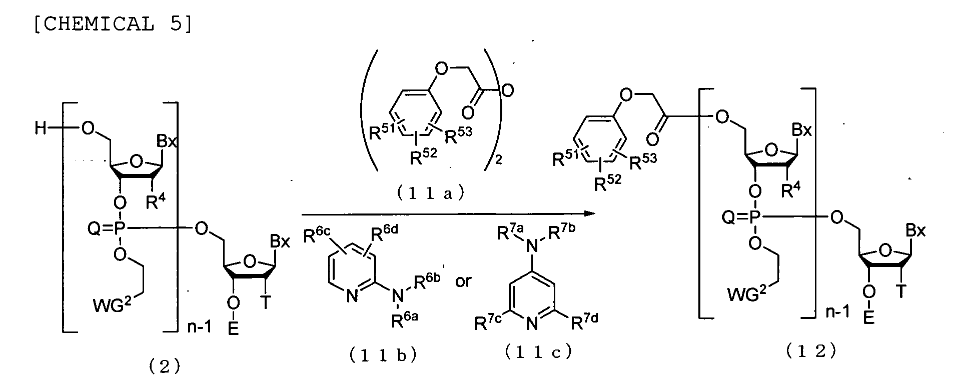 In the general formulae (2), (11a), (11b), (11c) and (12), each B_{X}, each Q, each R⁴ and each WG² independently have the same meanings as above. E, n, Q, R^{6a}, R^{6b}, R^{6c}, R^{6d}, R^{7a}, R^{7b}, R^{7c}, R^{7d}, R⁵¹, R⁵², R⁵³ and T have the same meanings as above. 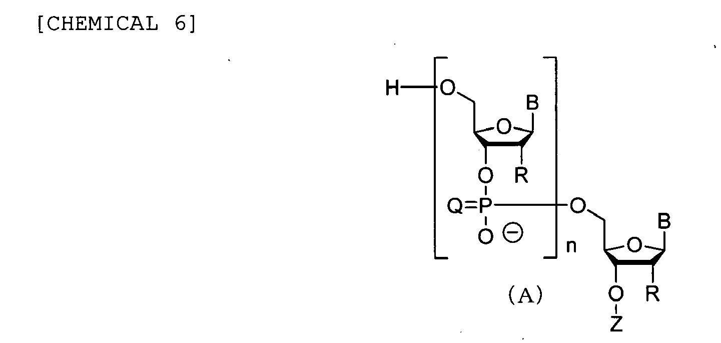 In the general formula (A), each Q and each R has the same meanings as above. n and Z have the same meanings as above. Each B independently represents a nucleobase or a modified form thereof.
The "nucleobase" of B is not particularly limited, and examples thereof may include pyrimidine bases such as cytosine, uracil and thymine, and purine bases such as adenine and guanine. The "modified form" of B is a group in which a nucleobase is substituted by an arbitrary substituent. Examples of the "substituent" related to the modified form of B may include halogen, acyl, alkyl, arylalkyl, alkoxy, alkoxyalkyl, hydroxy, amino, monoalkylamino, dialkylamino, carboxy, cyano and nitro. The modified form of B may be substituted by 1 to 3 of these substituents at arbitrary positions.
Examples of the "halogen", "acyl", "alkyl", "arylalkyl", "alkoxy", "alkoxyalkyl", "amino", "monoalkylamino" and "dialkylamino" related to the modified form of B may include the same ones as those related to the above-mentioned modified form of B_{X}. Examples of the "halogen", "alkoxy", "alkylamino" and "dialkylamino" related to R may include the same ones as those related to the above-mentioned modified form of B_{X}.
Examples of the "alkyl" moiety of the "alkylthio" and "alkoxyalkyloxy" related to R may include the same ones as those illustrated for the "alkyl" related to the above-mentioned modified form of B_{X}.
Examples of the "alkoxy" moiety of the "alkoxyalkyloxy" related to R may include the same ones as illustrated for the "alkoxy" related to the above-mentioned modified form of B_{X}.
Examples of the "alkenyl" moiety of the "alkenyloxy", "alkenylthio", "alkenylamino" and "dialkenylamino" related to R may include the same ones as illustrated for the "alkenyl" related to of the above-mentioned R⁴.
Examples of the "alkynyl" moiety of the "alkynylthio", "alkynylamino", "dialkynylamino" and "alkynyloxy" related to R may include the same ones as illustrated for the "alkynyl" related to the above-mentioned R⁴.
The "alkylamino", "alkenylamino" or "alkynylamino" related to R may be protected. The protecting group of the amino group is not particularly limited as long as it is a protecting group to be used as a protecting group of an amino group, and examples thereof may include trifluoroacetyl, benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. In particular, trifluoroacetyl is preferred.

Hereinafter, the present invention will be described in detail.

### BEST MODE FOR CARRYING OUT THE INVENTION

In the following production method, when raw materials have a substituent that affects the reaction (e.g., hydroxyl, amino and carboxy), the raw materials are used for reaction after being protected with a suitable protecting group according to a known method. After the reaction is completed, the protecting group can be removed by a known method such as catalytic reduction, alkali treatment, acid treatment or the like.

### I. Method for producing the oligonucleic acid (A)

The details of a method for producing the oligonucleic acid (A) are described below. 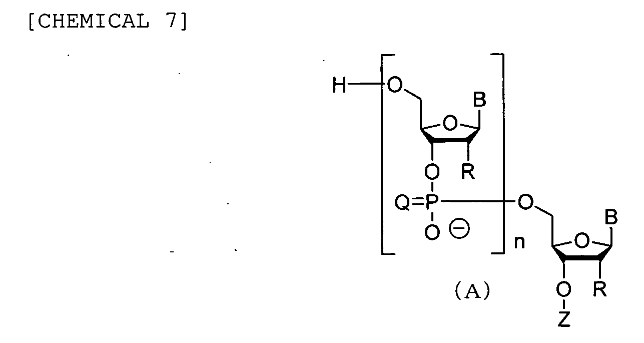 In the general formula (A), Q, each B, each Q and each R, independently have the same meanings as above. n and Z have the same meanings as above.
An oligo-RNA (A) can be produced by a known method, for example, it can be produced by condensing a nucleic acid monomer compound step by step in the 3' to 5' direction according to the following Steps A to H. Compounds and reagents to be used in the following step are not particularly limited as long as they are generally used in synthesis of oligo-RNAs or oligo-DNAs. In addition, all the steps can be performed by using an automated DNA synthesizer or manually as in the case of using conventional agents for synthesizing a nucleic acid. The use of an automated synthesizer is desirable from the point of view of the simplicity and ease of the method and the accuracy of the synthesis.

### (1) Step A:

Process for producing an (oligo)nucleic acid derivative represented by the following general formula (2) by removing the 5'-hydroxyl protecting group from an (oligo) nucleic acid derivative represented by the following general formula (1) by treating it with an acid. 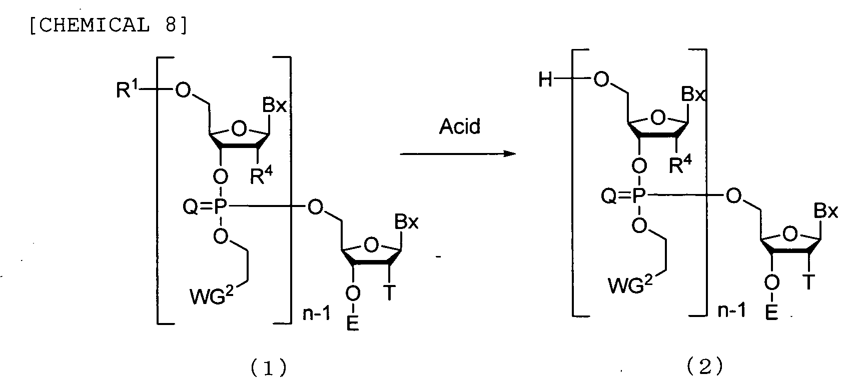 In the general formulae (1) and (2), n, E and T have the same meanings as above. Each B, each Q, each R⁴ and each WG² independently have the same meanings as above. Each R¹ represents a substituent represented by the following general formula (10). 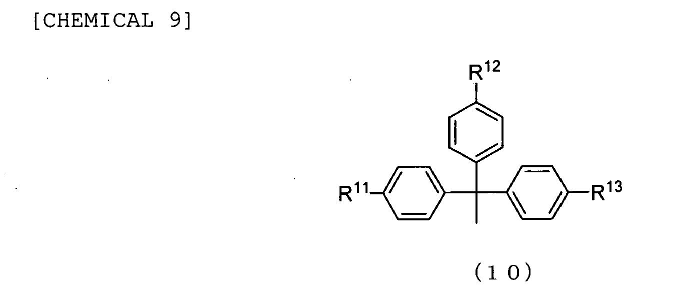 In the general formula (10), R¹¹, R¹² and R¹³ are the same or different and each represents hydrogen or alkoxy.
Examples of the "alkoxy" of R¹¹, R¹² and R¹³ may include the same ones as those illustrated for the "alkoxy" related to the above-mentioned modified form of B_{X}.
The step is performed by reacting an acid with a compound represented by the following formulae (6a) and (6b) (a nucleic acid derivative (1) wherein n is 1) which is attached to the solid support, or an oligo-RNA or an oligo-DNA produced by performing the operations of Steps A to D (oligonucleic acid derivative (1) wherein n is 2 to 100) which is attached to the solid support (hereinafter referred to as the "compound attached the solid support"). 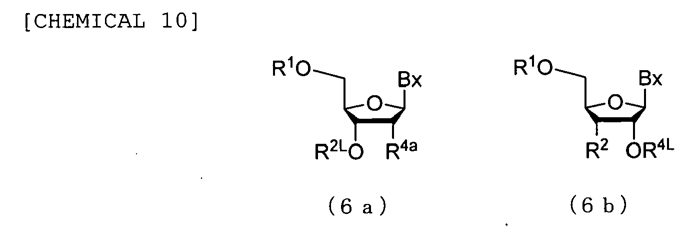 In the general formulae (6a) and (6b), B_{X} and R¹ have the same meanings as above. R^{2L} and R^{4L} represent substituent (4). R² represents acyloxy. R^{4a} represents H, acyloxy, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy or substituent (3).
Examples of "acyl" moiety of the "acyloxy" related to R² and R^{4a} may include acetyl, propionyl, butyryl, isobutyryl, benzoyl, 4-methoxybenzoyl, phenylacetyl, phenoxyacetyl, 4-tert-butylphenoxyacetyl, and 4-isopropylphenoxyacetyl.
Examples of the "halogen", "alkoxy", "alkylamino" or "dialkylamino" related to R^{4a} may include the same ones as those related to the above-mentioned modified form of B_{X}.
Examples of the "alkyl" moiety of "alkoxyalkyloxy" and "alkylthio" related to R^{4a} may include the same ones as those illustrated for the "alkyl" related to the above-mentioned modified form of B_{X}.
Examples of the "alkoxy" moiety of "alkoxyalkyloxy" related to R^{4a} may include the same ones as those illustrated for the "alkoxy" related to the above-mentioned modified form of B_{X}.
Examples of the "alkenyl" moiety of "alkenyloxy", "alkenylthio", "alkenylamino" and "dialkenylamino" related to R^{4a} may include the same ones as those illustrated for the "alkenyl" related to the above-mentioned R⁴.
Examples of the "alkynyl" moiety of "alkynyloxy", "alkynylthio", "alkynylamino" and "dialkynylamino" related to R^{4a} may include the same ones as those illustrated for the "alkynyl" related to the above-mentioned R⁴.
The "amino", "alkylamino", "alkenylamino" and "alkynylamino" of R^{4a} may be protected. The protecting group of the amino group is not particularly limited as long as it is a protecting group to be used as a protecting group of an amino group, and examples thereof may include trifluoroacetyl, benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. In particular, trifluoroacetyl is preferred.
Examples of the "solid support" may include a controlled-pore glass (CPG), an oxalyl-controlled pore glass (see, for example, Alul et al., Nucleic Acids Research, Vol. 19, 1527 (1991)), TentaGel support-amino polyethylene glycol derivatization support (see, for example, Wright et al., Tetrahedron Letters, Vol. 34, 3373 (1993)) and a copolymer of porous polystyrene and divinylbenzene.
Examples of the "linker" may include 3-aminopropyl, succinyl, 2,2'-diethanol sulfonyl and a long-chain alkylamino (LCAA). The nucleic acid derivatives (6a) and (6b), which are produced according to a known method or are commercially available, are attached to the solid support, and examples of a preferred embodiment are a nucleic acid derivative represented by the following general formula (7) or (8). 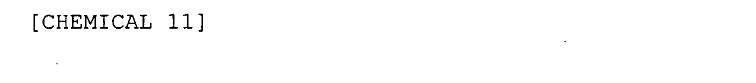 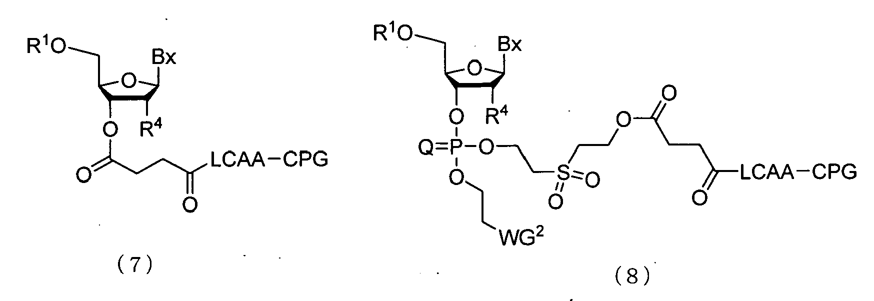 In the general formulae (7) and (8), B_{X}, Q, R¹, R⁴ and WG² have the same meanings as above.
The nucleic acid derivatives (7) and (8) wherein R⁴ is a substituent (3) can be produced from a phosphoramidite compound (B) according to a known method.
Examples of the "acid" to be used in the step may include trifluoroacetic anhydride, dichloroacetic acid, and trichloroacetic acid. The acid to be used in the step can be diluted with a suitable solvent to a concentration of 1 to 5%. The solvent is not specifically limited unless it is involved in the reaction, and it may include methylene chloride, acetonitrile, water and an arbitrary mixture thereof. The reaction temperature is preferably in the range of 20°C to 50°C. The reaction time depends on the kind of (oligo)nucleic acid derivative (1), the acid and the reaction temperature, and is preferably between 1 minute and 1 hour. The amount of the reagent to be used is preferably in the range of 0.8 to 100 mol per mol, and more preferably in the range of 1 to 10 mol per mol of the (oligo)nucleic acid derivative attached to the solid support.

### (2) Step B:

Process for producing an oligonucleic acid derivative represented by the following general formula (9) by condensing a nucleic acid monomer compound with the (oligo) nucleic acid derivative (2) produced by Step A using an activating agent. 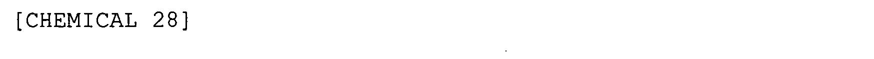 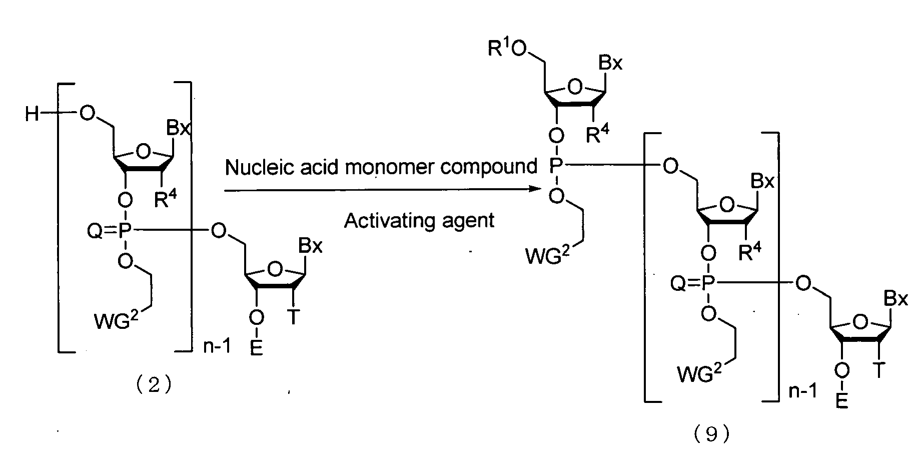 In the general formulae (2) and (9), each B_{X}, each Q, each R⁴ and each WG² independently have the same meanings as above. E, n, R¹ and T have the same meanings as above.
The step can be performed by reacting a nucleic acid monomer compound and an activating agent with an oligonucleic acid derivative attached to the solid support.
Examples of the "nucleic acid monomer compound" may include the phosphoramidite compound (B) and a nucleic acid derivative represented by the following general formula (C). 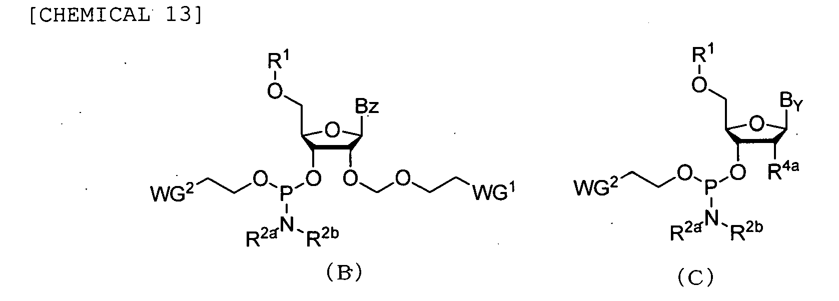 In the general formulae (B) and (C), B_{Y} and B_{Z} represent a nucleobase which may have protecting groups or a modified form thereof. R¹ and R⁴_{,} have the same meanings as above. R^{2a} and R^{2b} are the same or different and each represents alkyl, or R^{2a} and R^{2b}, together with the adjacent nitrogen atom, may form a 5- or 6-membered saturated cyclic amino group, the cyclic amino group optionally having one oxygen atom or one sulfur atom as a ring-forming member in addition to the adjacent nitrogen atom. WG¹ and WG² each represent an electron-withdrawing group.

The "nucleobase" related to B_{Z} is not particularly limited as long as it is a nucleobase to be used in the synthesis of a nucleic acid, and examples thereof may include pyrimidine bases such as cytosine, uracil and thymine, and purine bases such as adenine and guanine.
The "nucleobase" related to B_{Z} and B_{Y} may be protected, and particularly in the case of a nucleobase having an amino group such as adenine, guanine or cytosine, the amino group thereof is preferably protected. The "protecting group of the amino group" may include the same ones as those illustrated for the "protecting group of the amino group" related to the above-mentioned modified form of B_{X}. The "modified form" related to B_{Z} and B_{Y} is a group in which a nucleobase has been substituted by an arbitrary substituent. Examples of "substituent" for the "modified form" related to B_{Z} and B_{Y} may include halogen, acyl, alkyl, arylalkyl, alkoxy, alkoxyalkyl, hydroxyl, amino, monoalkylamino, dialkylamino, carboxy, cyano and nitro. The modified form of B_{Z} and B_{Y} may be substituted by 1 to 3 of these substituents at arbitrary positions.
Examples of the "halogen", "acyl", "alkyl", "arylalkyl", "alkoxy", "alkoxyalkyl", "monoalkylamino" or "dialkylamino" of the modified form related to B_{Z} and B_{Y} may include the same ones as those related to the above-mentioned modified form of B_{X}.

Examples of the "alkyl" related to R^{2a} and R^{2b} may include the same ones as those illustrated for the "alkyl" related to the above-mentioned modified form of B_{X}.
Examples of the "5- or 6-membered saturated cyclic amino group" related to R^{2a} and R^{2b} may include pyrrolidin-1-yl, piperidin-1-yl, morpholin-1-yl and thiomorpholin-1-yl.

Examples of the "activating agent" may include 1H-tetrazole, 5-ethylthiotetrazole, 5-benzylmercapto-1H-tetrazole, 4,5-dichloroimidazole, 4,5-dicyanoimidazole, benzotriazole triflate, imidazole triflate, pyridinium triflate, N,N-diisopropylethylamine and 2,4,6-collidine / N-methylimidazole. The reaction solvent to be used is not specifically limited unless it is involved in the reaction, and it may include, for example, acetonitrile and tetrahydrofuran (hereinafter abbreviated "THF"). The reaction temperature is preferably in the range of 20°C to 50°C. The reaction temperature depends on the kind of oligonucleic acid derivative (2), the kind of activating agent to be used and the reaction temperature, and is preferably between 1 minute and 1 hour. The amount of the agent to be used is preferably in the range of 1 to 100 mol per mol, and more preferably in the range of 1 to 10 mol per mol of the oligonucleic acid derivative attached to the solid support.

The phosphoramidite compound (B) is a phosphoramidite compound having an ether-type protecting group at the 2'-hydroxy position, which can be removed under neutral conditions. In addition, the phosphoramidite compound (B) is characterized by the facts that the condensation reaction proceeds in a shorter time and results in a better yield during the synthesis of oligo-RNAs when compared with a conventional phosphoramidite compound.

### (3) Step C:

Process for production of oligonucleic acid derivative represented by the following general formula (2), characterized by using a phenoxyacetic acid derivative anhydride represented by the following general formula (11a) as an acylating agent and a pyridine derivative represented by the following general formula (11b) or (11c) as the acylation reaction activator in a capping step for protecting the 5'-hydroxyl group of a ribose of an oligonucleic acid derivative represented by the following general formula (2) with an acyl group. 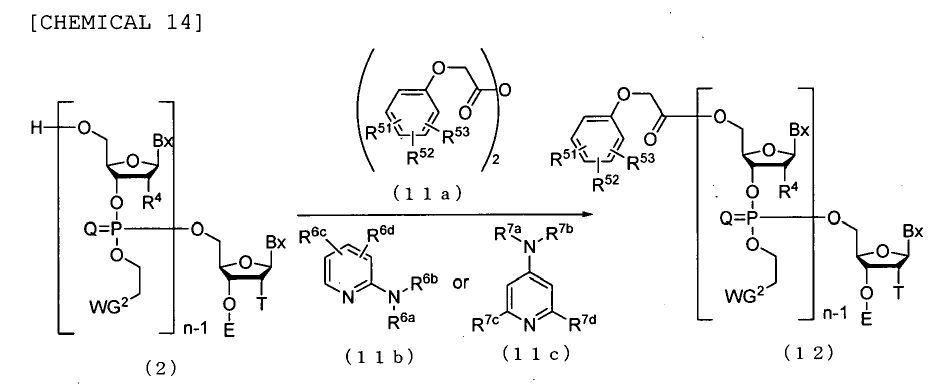 In the general formulae (2), (11a), (11b), (12) and (11c), each B_{X}, each Q, each R⁴ and each WG² independently have the same meanings as above. E, n, R^{6a}, R^{6b}, R^{6c}, R^{6d}, R^{7a}, R^{7b}, R^{7c}, R^{7d}, R⁵¹, R⁵², R⁵³ and T have the same meanings as above.
The step is a reaction for protecting the 5'-hydroxyl group of an unreacted (oligo)nucleotide (2) in Step B, and it can be performed by reacting a phenoxyacetic acid derivative anhydride (11a), a pyridine derivative (11b) or (11c) as the acylation reaction activator and a base with an unreacted (oligo) nucleotide (2) attached to the solid support.
The acylating agent to be used can be diluted with a suitable solvent to a concentration of 0.05 to 1 M. The amount of the acylating agent to be used is preferably in the range of 0.8 to 100 mol per mol, and more preferably in the range of 10 to 30 mol per mol of the oligonucleic acid derivative (2) attached to the solid support. The amount of the acylation reaction activator to be used may be in the range of 0.8 to 50 mol per mol, and preferably in the range of 1 to 10 mol per mol of the phenoxyacetic anhydride derivative (11a). In addition, for example, pyridine, 2,6-lutidine, 2,4,6-collidine, or a mixture thereof can be used, if necessary, as a scavenger of the phenoxyacetic acid derivative which is a by-product of this step. Among these, 2, 6-lutidine is preferable. The amount of the base to be used depends on the kind of oligonucleic acid derivative (2) attached to the solid support and on the phenoxyacetic anhydride derivative (11a), and may be in the range of 0.8 to 100 mol per mol, and preferably in the range of 1 to 20 mol per mol of the phenoxyacetic anhydride derivative (11a). The solvent to be used in the reaction is not specifically limited unless it is involved in the reaction, and it may include, for example, methylene chloride, acetonitrile, THF and mixtures thereof. The reaction temperature is preferably in the range of 20°C to 50°C. The reaction time depends on the kind of oligonucleic acid derivative (2), the acylating agent to be used, the acylation reaction activator to be used, the base and the reaction temperature, and may be between 1 and 30 min.

### (4) Step D:

Process for converting a phosphite group into a phosphate group or thiophosphate group by reacting an oxidizing agent with the oligonucleic acid derivative (9) produced in Step B. 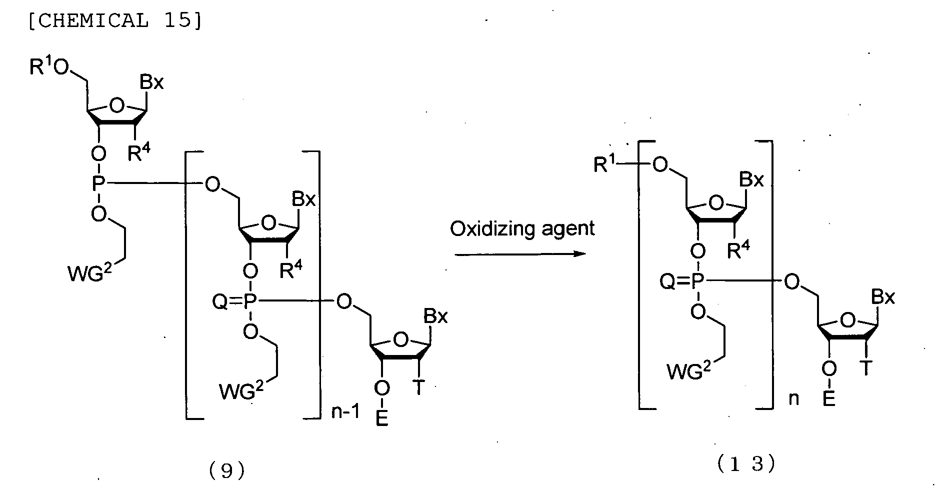 In the general formulae (9) and (15), each B_{X}, each Q, each R⁴ and each WG² independently have the same meanings as above. E, n, R¹ and T have the same meanings as above.
The step is a reaction for converting trivalent phosphorus to pentavalent phosphorus by using an oxidizing agent, and it can be performed by reacting an oxidizing agent with an oligonucleic acid derivative attached to the solid support.
When phosphorus is oxidized by oxygen, examples of the "oxidizing agent" may include iodine and tert-butylhydroperoxide. In addition, the oxidizing agent to be used can be diluted with a suitable solvent to a concentration of 0.05 to 2 M. The reaction solvent to be used is not specifically limited unless it is involved in the reaction, and it may include, for example, pyridine, tetrahydrofuran, water and mixtures thereof. For example, iodine / water / pyridine - THF, iodine / pyridine - acetic acid and a peroxidation agent (tert-butylhydroperoxide / methylene chloride and the like) can be used.
In addition, when phosphorus is oxidized by sulfur, examples of the "oxidizing agent" may include sulfur, Beaucage reagent (3H-1,2-benzodithiol-3-one-1,1-dioxide) and 3-amino-1,2,4-dithiazole-5-thione (ADTT).
The oxidizing agent to be used can be diluted with a suitable solvent in the range of a concentration of 0.01 to 2 M. The reaction solvent to be used is not specifically limited unless it is involved in the reaction, and it may include, for example, methylene chloride, acetonitrile, pyridine and mixtures thereof. The reaction temperature is preferably in the range of 20°C to 50°C. The reaction time depends on the kind of oligonucleic acid derivative (9), the oxidizing agent and the reaction temperature, and is preferably between 1 and 30 minutes. The amount of the oxidizing agent to be used is preferably in the range of 0.8-100 mol per mol, and more preferably in the range of 1-50 mol per mol of the oligonucleic acid derivative attached to the solid support.

### (5) Step E:

Process for cleaving the oligonucleic acid derivative (13) produced by Step D from the solid support, and then removing the protecting groups of each nucleobase and each phosphate group. 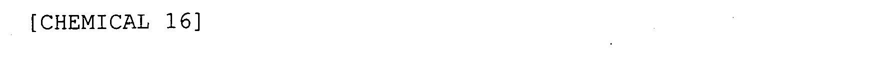 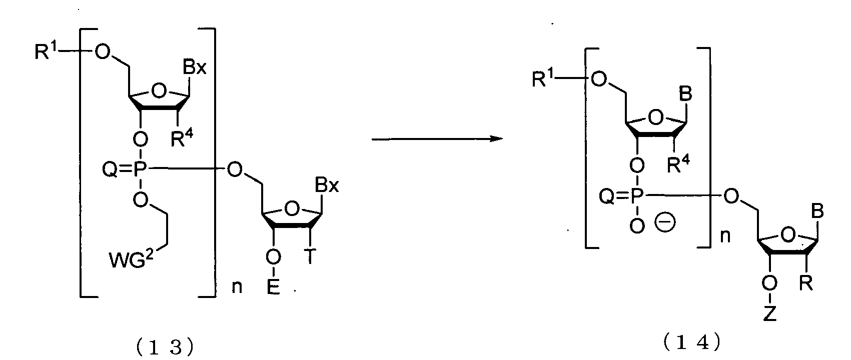 In the general formulae (13) and (14), each B, each B_{X}, each Q, each R⁴ and each WG² independently have the same meanings as above. E, n, R, R¹, T and Z have the same meanings as above.
The cleavage step is a reaction for cleaving an oligoribonucleic acid having a desired chain length from the solid support and linker with a cleaving agent, and is performed by adding a cleaving agent to the solid support to which is attached an oligonucleotide having a desired chain length.
In the step, the protecting group of a nucleobase can be removed. Examples of the "cleaving agent" may include concentrated aqueous ammonia and methylamine. The cleaving agent to be used in the step may be diluted by, for example, water, methanol, ethanol, isopropyl alcohol, acetonitrile, THF and mixtures thereof. Among them, ethanol is preferred. The reaction temperature may be in the range of 15°C to 75°C, is preferably in the range of 15°C to 30°C, and is more preferably in the range of 18°C to 25°C. The reaction time for deprotection depends on the kind of oligonucleic acid derivative (9), the oxidizing agent and the reaction temperature, and may be in the range of 10 minutes to 30 hours, is preferably in the range of 30 minutes to 24 hours, and is more preferably in the range of 1 to 4 hours. The concentration of ammonium hydroxide in the solution to be used for deprotection may be 20 to 30% by weight, is preferably 25 to 30% by weight, and is more preferably 28 to 30% by weight. The amount of the ammonium hydroxide to be used may be in the range of 1 to 100 mol per mol, and preferably 10 to 50 mol per mol of the oligonucleic acid derivative (13) attached to the solid support.

### (6) Step F:

Process for producing the oligonucleic acid derivative represented by the following general formula (15) by reacting a reagent for removing the 2'-hydroxyl protecting group of each ribose in the oligonucleic acid derivative (14) produced in Step E. 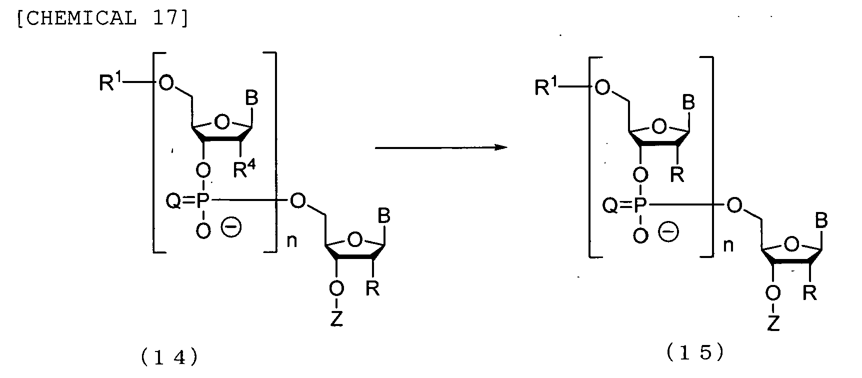 In the general formulae (14) and (15), each B, each Q, each R and each R⁴ independently have the same meanings as above. n, R¹ and Z have the same meanings as above.
The step can be performed by reacting an agent for removing the 2'-hydroxyl protecting group with the oligonucleic acid derivative (14). The step for removing the 2'-hydroxyl protecting group is performed by reacting an agent for removing the 2'-hydroxyl protecting group such as tetrabutylammonium fluoride, and triethylamine/trihydrogen fluoride. The amount of the agent for removing the 2'-hydroxyl protecting group may be in the range of 1 to 500 mol per mol, and is preferably in the range of 5 to 10 mol per mol of the protecting group to be removed. The solvent to be used is not specifically limited unless it is involved in the reaction, and it may include, for example, THF, N-methylpyrrolidone, pyridine, dimethylsulfoxide and mixtures thereof. The solvent to be used the reaction may be in the range of 0.8 to 100 mol per mol, and preferably in the range of 1 to 10 mol per mol of the agent for removing the 2'-hydroxyl protecting group. The reaction temperature is preferably in the range of 20°C to 80°C. The reaction time depends on the kind of oligonucleic acid derivative (14), the agent for removing the 2'-hydroxyl protecting group to be used and the reaction temperature, and is preferably in the range of 1 to 100 hours.
In addition, a nitroalkane, alkylamine, amidine, thiol, thiol derivative or a mixture thereof can be added, if necessary, as a scavenger of acrylonitrile, which is a by-product of the step.
Examples of the "nitroalkane" may include straight nitroalkanes having 1 to 6 carbon atoms. Specifically, the nitroalkane may include, for example, nitromethane.
Examples of the "alkylamine" may include straight alkylamine having 1 to 6 carbon atoms. Specifically, the "alkylamine" may include, for example, methylamine, ethylamine, n-propylamine, n-butylamine, n-pentylamine and n-hexylamine.
Examples of the "amidine" may include benzamidine and formamidine.
Examples of the "thiol" may include straight thiols having 1 to 6 carbon atoms. Specifically, the "thiol" may include, for example, methanethiol, ethanethiol, 1-propanethiol, 1-butanethiol, 1-pentanethiol and 1-hexanethiol.
Examples of the "thiol derivative" may include alcohols and ethers having the same or different straight alkylthiol having 1 to 6 carbon atoms. Specifically, the thiol derivative may include, for example, 2-mercaptoethanol, 4-mercapto-1-butanol, 6-mercapto-1-hexanol, mercaptomethyl ether, 2-mercaptoethyl ether, 3-mercaptopropyl ether, 4-mercaptobutyl ether, 5-mercaptopentyl ether and 6-mercaptohexyl ether.
The amount of the scavenger of acrylonitrile to be used depends on the kind of oligonucleic acid derivative (14), and may be in the range of 0.8 to 500 mol per mol, and is preferably in the range of 1 to 10 mol per mol of 2-cyanoethoxymethyl substituting the 2'-hydroxyl group of each ribose of the oligonucleic acid derivative (14).

### (7) Step G:

Process for removing the 5'-hydroxyl group of the oligonucleic acid derivative (15). 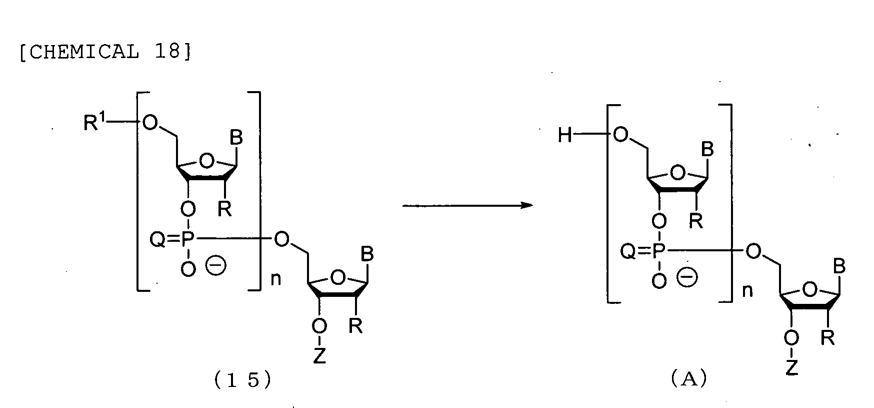 In the general formulae (15) and (A), each B, each Q and each R independently have the same meanings as above. n, R¹ and Z have the same meanings as above.
The step is a reaction for finally removing the protecting group of the 5'-hydroxyl group of the oligonucleotide (15), and it can be performed by reacting an acid with the oligo-RNA cleaved from the solid support.
Examples of the "acid" to be used in the step may include trichloroacetic acid, dichloroacetic acid and acetic acid. The acid can be diluted with a suitable solvent for use in the step. The solvent is not specifically limited unless it is involved in the reaction, and it may include, for example, methylene chloride, acetonitrile, water, a buffer whose pH is in the range of 2 to 5, and mixtures thereof.
Examples of the "buffer solution" may include an acetate buffer. The reaction temperature is preferably in the range of 20°C to 50°C. The reaction time for deprotection depends on the kind of oligonucleic acid derivative (15), the acid and the reaction temperature, and may be in the range of 1 minute to 1 hour. The amount of the reagent to be used may be in the range of 0.8 to 100 mol per mol, and is preferably in the range of 1 to 10 mol per mol of the oligonucleic acid derivative attached to the solid support.

### (8) Step H:

Process for isolating and purifying the oligoribonucleic acid (A) produced by Step G.
The step of isolating and purifying is a step for isolating and purifying the desired oligo-RNA from the above reaction mixture by a known method for isolating and purifying which may include, for example, extraction, concentration, neutralization, filtration, centrifugal separation, recrystallization, reverse-phase column chromatography (C₈ to C₁₈), the use of a reverse-phase cartridge column (C₈ to C₁₈), cation-exchange column chromatography, anion-exchange column chromatography, gel filtration column chromatography, high performance liquid chromatography, dialysis, ultrafiltration and combinations thereof.
Examples of the eluent may include acetonitrile, methanol, ethanol, isopropyl alcohol, water and a mixed solvent at an arbitrary ratio. In this case, for example, the pH of the solution can be controlled to be in the range of 1 to 9 by adding sodium phosphate, potassium phosphate, sodium chloride, potassium chloride, ammonium acetate, triethylammonium acetate, sodium acetate, potassium acetate, tris-hydrochloric acid or ethylenediaminetetraacetic acid as an additive at a concentration of 1 mM to 2 M.

An oligoribonucleic acid (A) of a desired chain length can be produced by repeated operation of steps A to D.
Additionally, in process B of the method for producing the oligonucleic acid mentioned above, it is possible to produce an oligonucleic acid (A) in which at least one of the Rs is a hydroxyl group by using the phosphoramidite compound (B) at least once as a nucleic acid monomer compound. Furthermore, in process B of the method for producing the oligonucleic acid mentioned above, it is possible to produce the oligonucleic acid (A) in which all Rs of the oligonucleic acid (A) are hydroxyl groups, by using the phosphoramidite compound (B) entirely as a nucleic acid monomer compound.

### II. A method of producing the phosphoramidite compound (B)

The phosphoramidite compound (B) can be produced as follows. In the following production method, when raw materials have a substituent that affects the reaction (e.g., hydroxy, amino and carboxy), the raw materials are used for reaction after being protected with a suitable protecting group according to a known method. The protecting groups can finally be removed by well-known methods such as catalytic reduction, alkali treatment or acid treatment. The phosphoramidite compounds (B) can be produced from a known compound or an intermediate which can easily be produced through the following Steps a to h, for example. The method of producing the phosphoramidite compound according to the present invention is described in detail below.

### (1) Step a:

Process for producing a nucleoside derivative represented by the following general formulae (17) and (17'), wherein an ether-type protecting group which can be removed under neutral conditions is introduced at the 2'-hydroxyl position by allowing an alkylating reagent to act on a nucleoside derivative represented by the following general formula (16). 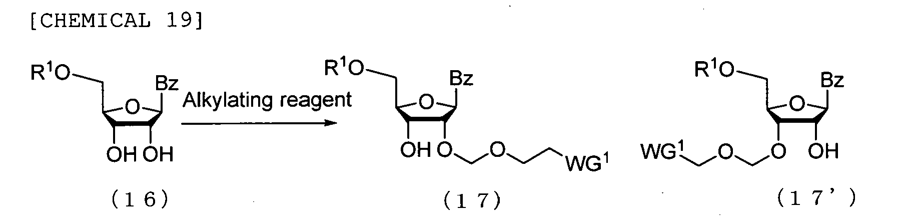 In the general formulae (16), (17) and (17'), B_{Z}, R¹ and WG¹ have the same meanings as above.
Examples of the "alkylating regent" may include an ether compound represented by the following general formula (18). 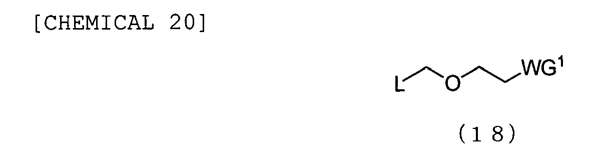 In the general formula (18), L represents halogen, an arylthio group, an alkylsulfoxide group or an alkylthio group. WG¹ has the same meanings as above.
Examples of the "halogen", the "aryl" moiety of the "arylthio group", the "alkyl" moiety of the "alkylsulfoxide group", and the "alkylthio group" related to L may include the same ones as those related to the above-mentioned modified form of B_{X}.
Specific examples of the ether compound (18) may include the following compounds 1 or 2:
1. Chloromethyl 2-cyanoethyl ether
2. 2-cyanoethyl methylthiomethyl ether
The ether compound (18) is a new alkylating reagent which can introduce an ether-type substituent, which is removable under neutral conditions, at the 2'-hydroxyl position under basic conditions, and which is useful as a reagent for producing the phosphoramidite compound (B).

The ether compounds (18) can be produced by the following Steps 1 to 4.

### Step 1:

Process for producing a compound represented by the following general formula (20) by alkylthiomethylating an alcohol compound represented by the following general formula (19). 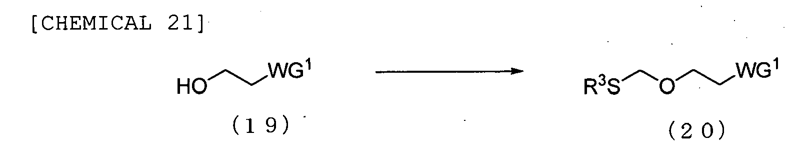 In the general formulae (19) and (20), WG¹ has the same meanings as above. R³ represents alkyl or aryl.
Compound (20) is the ether compound (18) wherein L is an alkylthio group.
Examples of "alkyl" related to R³ may include the same ones as those illustrated for the "alkyl" related to the above-mentioned modified form of B_{X}. When R³ is methyl, examples of the "alkylthiomethylating reagent" may include a mixed solvent containing dimethylsulfoxide, acetic anhydride and acetic acid. The amount of dimethylsulfoxide to be used may be in the range of 10 to 200 mol per mol, and is preferably in the range of 20 to 100 mol per mol of compound (19). The amount of acetic acid to be used may be in the range of 10 to 150 mol per mol, and is preferably in the range of 20 to 100 mol per mol of compound (19). The amount of acetic anhydride to be used may be in the range of 10 to 150 mol per mol, and is preferably in the range of 20 to 100 mol per mol of compound (19). The reaction temperature is preferably in the range of 0°C to 100°C. The reaction time depends on the kind of raw materials and the reaction temperature, and is preferably between 1 and 48 hours.

### Step 2:

Process for producing a compound represented by the following general formula (21) by halogenating compound (20). 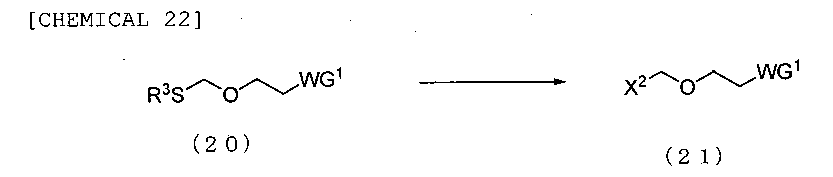 In the general formulae (20) and (21), WG¹ and R³ have the same meanings as above. X² represents halogen.
Compound (21) is a compound wherein L of the ether compound (18) is halogen.
Examples of the "halogen" related to X² may include the same ones as those illustrated for the "halogen" related to the above-mentioned modified form of B_{X}.
The step can be carried out by well-known methods (e.g., T. Benneche et al., Synthesis 762 (1983)). The solvent to be used is not specifically limited unless it is involved in the reaction, and it may include, for example, a halogenated hydrocarbon such as methylene chloride, chloroform, carbon tetrachloride and 1,2-dichloroethane. Examples of the "halogenating agent" may include sulfuryl chloride and phosphorus oxychloride. The amount of the halogenating agent to be used may suitably be in the range of 0.8 to 20 mol per mol, and is preferably in the range of 1 to 10 mol per mol of compound (20). The reaction temperature is preferably in the range of 0°C to 100°C. The reaction time depends on the kind of raw materials and the reaction temperature, and is preferably between 30 minutes and 24 hours.

### Step 3:

Process for producing a compound represented by the following general formula (22) by arylthiolating compound (21). 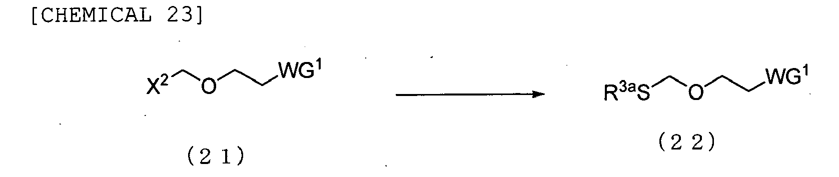 In the general formulae (21) and (22), WG¹ and X² have the same meanings as above. R^{3a} represents aryl.
Compound (22) is a compound (18) wherein L is an arylthio group. Examples of the "aryl" related to R^{3a} may include the same ones as those illustrated for the "aryl" related to the above-mentioned modified form of B_{X}.
The step can be carried out by a known method. The solvent to be used is not specifically limited unless it is involved in the reaction, and it may include, for example, methylene chloride and acetonitrile.
Examples of the "arylthiolating reagent" may include thiophenol or 4-methyl benzenethiol. The amount of the arylthiolating reagent to be used may be in the range of 0.8 to 20 mol per mol, and is preferably in the range of 1 to 5 mol per mol of compound (21). The reaction temperature is preferably in the range of 0°C to 100°C. The reaction time depends on the kind of raw materials and the reaction temperature, and is preferably between 1 and 48 hours.

### Step 4:

Process for producing a compound represented by the following general formula (23) by oxidizing compound (20). 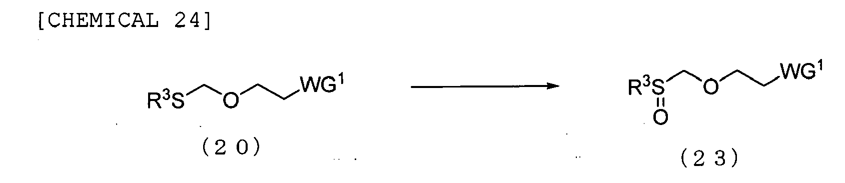 In the general formulae (20) and (23), WG¹ and R³ have the same meanings as above.
The compound (23) is a compound (18) wherein L is an alkylsulfoxide group. Examples of the "alkyl" related to R³ may include the same ones as those illustrated for the "alkyl" related to the above-mentioned phosphoramidite compound (B).
The step can be carried out by a known method. The solvent to be used is not specifically limited unless it is involved in the reaction, and it may include, for example, methylene chloride, chloroform and methanol. Examples of the "oxidizing agent" may include metachloroperbenzoic acid, metaperiodate salt and hydrogen peroxide. The amount of the oxidizing agent to be used may be in the range of 0.8 to 10 mol per mol, and is preferably in the range of 1 to 2 mol per mol of compound (20). The reaction temperature is preferably in the range of 0°C to 100°C. The reaction time depends on the kind of raw materials and the reaction temperature, and is preferably between 1 and 48 hours.

When compound (21) is used as the alkylating agent, the step can be performed as follows.
The step can be performed by reacting the alkylating agent and a base with ribonucleic acid derivative (16), which is commercially available or is synthesized according to a known method. The solvent to be used is not specifically limited unless it is involved in the reaction, and it may include, for example, a halogenated hydrocarbon such as methylene chloride, chloroform, carbon tetrachloride and 1,2-dichloroethane. The amount of the alkylating agent to be used may be in the range of 0.8 to 20 mol per mol, and is preferably in the range of 1 to 10 mol per mol of the ribonucleic acid derivative (16).
In the step, the alkylating agent may, if necessary, be reacted with the intermediate produced by reacting a metal reagent and a base with ribonucleic acid derivative (16). Examples of the "metal reagent" may include dibutylstannyl dichloride. The amount of the metal reagent to be used may be in the range of 0.8 to 20 mol per mol, and is preferably in the range of 1 to 10 mol per mol of the ribonucleic acid derivative (16). Examples of the "base" may include organic bases such as pyridine, 2,6-dimethylpyridine, 2,4,6-trimethylpyridine, N-methylimidazole, triethylamine, tributylamine, N,N-diisopropylethylamine and 1,8-diazabicyclo[5.4.0]-7-undecene. The amount of the base to be used may be in the range of 0.8 to 20 mol per mol, and is preferably in the range of 1 to 10 mol per mol of the ribonucleic acid derivative (16) . The reaction temperature is preferably in the range of 0°C to 120°C. The reaction time depends on the kind of raw materials and the reaction temperature, and is preferably between 30 minutes and 24 hours.

When compound (20) or (22) is used as the alkylating reagent, the step can be performed as follows.
The step can be performed according to a known method (e.g., M. Matteucci, Tetrahedron Letters, Vol. 31, 2385 (1990)) by reacting the alkylating reagent, an acid and a reagent for halogenating the sulfur atom on ribonucleic acid derivative (16), which is commercially available or is synthesized according to a known method. The amount of the alkylating reagent to be used may be in the range of 0.8 to 5 mol per mol, and is preferably in the range of 1 to 3 mol per mol of the ribonucleic acid derivative (16). Examples of the "acid" may include trifluoromethanesulfonic acid, silver trifluoromethanesulfonate and trimethylsilyl trifluoromethanesulfonate. The amount of the acid to be used may be in the range of 0.01 to 20 mol per mol, and is preferably in the range of 0.02 to 10 mol per mol of the ribonucleic acid derivative (16). The solvent to be used is not specifically limited unless it is involved in the reaction, and it may include, for example, methylene chloride, chloroform, carbon tetrachloride, 1,2-dichloroethane, benzene, toluene, xylene, THF, acetonitrile and mixtures thereof. Examples of the "reagent for halogenating a sulfur atom" to be used in the step may include N-bromosuccinimide (NBS) and N-iodosuccinimide (NIS). The amount of the reagent for halogenating a sulfur atom to be used may be in the range of 0.8 to 10 mol per mol, and is preferably in the range of 1 to 5 mol per mol of the ribonucleic acid derivative (12). The reaction temperature is preferably in the range of -78°C to 30°C. The reaction time depends depending on the kind of raw materials and the reaction temperature, and is preferably between 5 minutes and 5 hours.

When compound (23) is used as the alkylating reagent, the step can be performed as follows.
The step can be performed by reacting the alkylating reagent, an acid anhydride and a base with ribonucleic acid derivative (16), which is commercially available or is synthesized according to a known method. The amount of the alkylating reagent to be used may be in the range of 0.8 to 5 mol per mol, and is preferably in the range of 1 to 3 mol per mol of the ribonucleic acid derivative (16). Examples of the "acid anhydride" may include trifluoromethanesulfonic anhydride and acetic anhydride. The amount of the acid anhydride to be used may be in the range of 0.01 to 20 mol per mol, and is preferably in the range of 0.02 to 10 mol per mol of the ribonucleic acid derivative (16). Examples of the "base" may include tetramethylurea or collidine. The amount of the base to be used may be in the range of 0.01 to 20 mol per mol, and is preferably in the range of 0.02 to 10 mol per mol of the ribonucleic acid derivative (16). The solvent to be used is not specifically limited unless it is involved in the reaction, and it may include, for example, methylene chloride, chloroform, carbon tetrachloride, 1,2- dichloroethane and mixtures thereof. The reaction temperature is preferably in the range of -78°C to 30°C. The reaction time depends on the kind of raw materials and the reaction temperature, and is preferably between 5 minutes and 24 hours.

### (2) Step b:

Process for isolating and purifying the nucleoside derivative (17) produced by Step a;
In the step, the nucleoside derivative can be isolated and purified from the mixture produced by step a by using a standard separation and purification technique such as thin-layer chromatography, silica gel column chromatography or the like.

### (3) Step c:

Process for producing a ribonucleic acid compound represented by the following general formula (25), wherein an ether-type protecting group which can be removed under neutral conditions is introduced at the 2'-hydroxyl position, by allowing an alkylating reagent to act on a ribonucleic acid derivative represented by the following general formula (24), being independent of Step b. 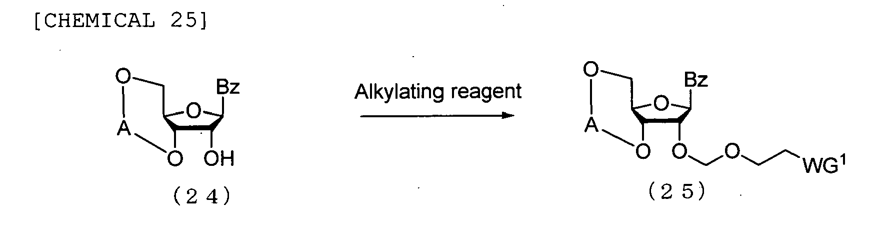 In the general formulae (24) and (25), B_{Z} and WG¹ have the same meanings as above. A represents a silicon substituent represented by the following general formula (26a) or (26b). 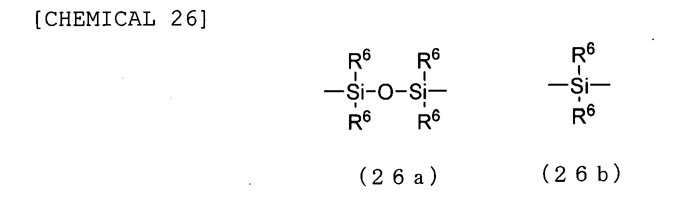 In the general formulae (26a) and (26b), R⁶ represents alkyl.
Examples of the "alkyl" of R⁶ may include the same alkyl as that of the phosphoramidite compound (B).
Examples of the "alkylating reagent" may include the same ones as those illustrated in the above description.

When compound (21) is used as the alkylating agent, the step can be performed as follows.
The step can be performed by reacting the alkylating agent and a base with a Ribonucleic acid derivative (24), which is commercially available or is synthesized according to a known method. The solvent to be used is not specifically limited unless it is involved in the reaction, and it may include, for example, halogenated hydrocarbon such as methylene chloride, chloroform, carbon tetrachloride and 1,2-dichloroethane. The amount of the alkylating agent to be used may be in the range of 0.8 to 20 mol per mol, and is preferably in the range of 1 to 10 mol per mol of the ribonucleic acid derivative (24). In the step, the alkylating agent may, if necessary, be reacted with the intermediate produced by reaction of a metal reagent and a base with the ribonucleic acid derivative (24).
Examples of the "metal reagent" may include dibutylstannyl dichloride and tert-butylmagnesium chloride. The amount of the metal reagent to be used may be in the range of 0.8 to 20 mol per mol, and is preferably in the range of 1 to 10 mol per mol of the ribonucleic acid derivative (24).
Examples of the "base" may include organic bases such as pyridine, 2,6-dimethylpyridine, 2,4,6-trimethylpyridine, N-methylimidazole, triethylamine, tributylamine, N,N-diisopropylethylamine and 1,8-diazabicyclo[5.4.0]-7-undecene. The amount of the base to be used may be in the range of 0.8 to 20 mol per mol, and is preferably in the range of 1 to 10 mol per mol of the ribonucleic acid derivative (24). The reaction temperature is preferably in the range of 0°C to 120°C. The reaction time depends on the kind of raw materials and the reaction temperature, and is preferably between 30 minutes and 24 hours.

When compound (20) or (22) is used as the alkylating agent, the step can be performed as follows.
The step can be performed according to a known method (for example, M. Matteucci, Tetrahedron Letters, Vol. 31, 2385 (1990)) by reacting the alkylating agent, an acid and a reagent for halogenating the sulfur atom with a ribonucleic acid derivative (24), which is commercially available or is synthesized by a known method. The amount of the alkylating agent to be used may be in the range of 0.8 to 5 mol per mol, and is preferably in the range of 1 to 3 mol per mol of the ribonucleic acid derivative (24). Examples of the "acid" may include trifluoromethanesulfonic acid, silver trifluoromethanesulfonate and trimethylsilyl trifluoromethanesulfonate. The amount of the acid to be used may be in the range of 0.01 to 20 mol per mol, and is preferably 0.02 to 10 mol per mol of the ribonucleic acid derivative (24). The solvent to be used is not specifically limited unless it is involved in the reaction, and it may include, for example, methylene chloride, chloroform, carbon tetrachloride, 1,2- dichloroethane, benzene, toluene, xylene, THF, acetonitrile and mixtures thereof. Examples of the "reagent for halogenating a sulfur atom" to be used in the step may include N-bromosuccinimide (NBS) and N-iodosuccinimide (NIS). The amount of the reagent for halogenating a sulfur atom to be used may be in the range of 0.8 to 10 mol per mol, and is preferably in the range of 1 to 5 mol per mol of the ribonucleic acid derivative (24). The reaction temperature is preferably in the range of -78°C to 30°C. The reaction time depends on the kind of raw materials and the reaction temperature, and is preferably between 5 minutes and 5 hours.

When compound (23) is used as the alkylating reagent, the step can be performed as follows.
The step can be performed by reacting the alkylating reagent, an acid anhydride and a base with ribonucleic acid derivative (24), which is commercially available or is synthesized according to a known method. The amount of the alkylating reagent to be used may be in the range of 0.8 to 5 mol per mol, and is preferably in the range of 1 to 3 mol per mol of the ribonucleic acid derivative (24). Examples of the "acid anhydride" may include trifluoromethanesulfonic anhydride and acetic anhydride. The amount of the acid anhydride to be used may be in the range of 0.01 to 20 mol per mol, and is preferably in the range of 0.02 to 10 mol per mol of the ribonucleic acid derivative (24). Examples of the "base" may include tetramethylurea or collidine. The amount of the base to be used may be in the range of 0.01 to 20 mol per mol, and is preferably in the range of 0.02 to 10 mol per mol of the ribonucleic acid derivative (24). The solvent to be used is not specifically limited unless it is involved in the reaction, and it may include, for example, methylene chloride, chloroform, carbon tetrachloride, 1,2-dichloroethane or mixtures thereof. The reaction temperature is preferably in the range of -78°C to 30°C. The reaction time depends on the kind of raw materials and the reaction temperature, and is preferably between 5 minutes and 24 hours.

### (4) Step d:

Process for producing a ribonucleic acid derivative represented by the following general formula (27) by allowing dimethylsulfoxide, acetic acid and acetic anhydride to act on the ribonucleic acid derivative (24), being independent of Steps a to c. 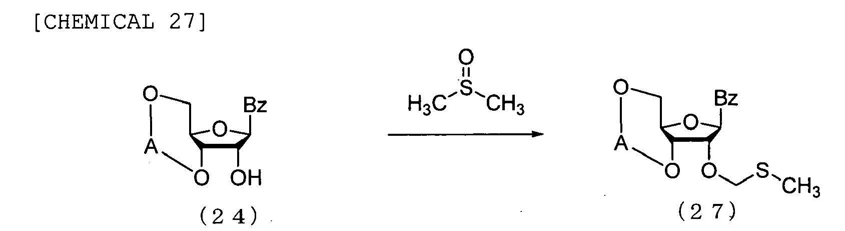 In the general formulae (24) and (27), A and B_{Z} have the same meanings as above.
The step can be performed by reacting dimethylsulfoxide, acetic acid and acetic anhydride with a ribonucleic acid derivative (24), which is commercially available or is synthesized according to a known method. The amount of the dimethylsulfoxide to be used may be in the range of 10 to 200 mol per mol, and is preferably in the range of 20 to 100 mol per mol of the ribonucleic acid derivative (24). The amount of the acetic acid to be used may be in the range of 10 to 150 mol per mol, and is preferably in the range of 20 to 100 mol per mol of the ribonucleic acid derivative (24). The amount of the acetic anhydride to be used may be in the range of 10 to 150 mol per mol, and is preferably in the range of 20 to 100 mol per mol of the ribonucleic acid derivative (24). The reaction temperature is preferably in the range of 10°C to 50°C. The reaction time depends on the kind of raw materials and the reaction temperature, and is preferably between 30 minutes and 24 hours.

### (5) Step e:

Process for producing a ribonucleic acid derivative represented by the following general formula (25), wherein an ether-type protecting group which can be removed under neutral conditions is introduced at the 2'-hydroxyl position, by allowing an alcohol compound represented by the following general formula (28), an acid and a reagent for halogenating a sulfur atom to act on a ribonucleic acid derivative (27) produced by Step d. 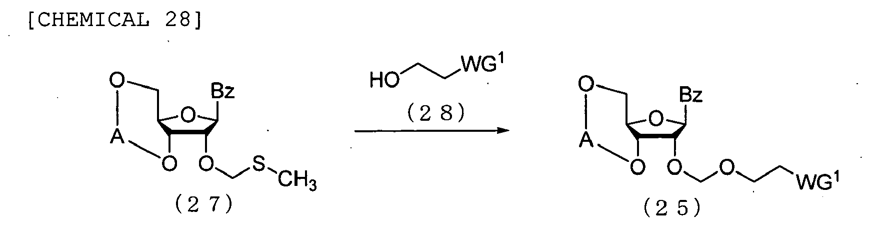 In the general formulae (25), (27) and (28), A, B_{Z} and WG¹ have the same meanings as above.
The step can be performed by reacting the alcohol compound (28), an acid and a reagent for halogenating the sulfur atom with the ribonucleic acid derivative (27) according to a known method. The solvent to be used is not specifically limited unless it is involved in the reaction, and it may include, for example, methylene chloride, chloroform, carbon tetrachloride, 1,2-dichloroethane, benzene, toluene, xylene, THF, acetonitrile and mixtures thereof. The amount of the alcohol compound (28) to be used may be in the range of 0.8 to 20 mol per mol, and is preferably in the range of 1 to 10 mol per mol of the ribonucleic acid derivative (27). Examples of the "acid" may include trifluoromethanesulfonic acid, silver trifluoromethanesulfonate and trimethylsilyl trifluoromethanesulfonate. Examples of the "reagent for halogenating a sulfur atom" may include N-bromosuccinimide (NBS) and N-iodosuccinimide (NIS). The amount of the reagent for halogenating a sulfur atom to be used may be in the range of 0.1 to 20 mol per mol, and is preferably in the range of 0.2 to 10 mol per mol of the ribonucleic acid derivative (27). The reaction temperature is preferably in the range of -100°C to 20°C. The reaction time depends on the kind of raw materials and the reaction temperature, and is preferably between 5 minutes and 12 hours.

### (6) Step f:

Process for producing a ribonucleic acid derivative represented by the following general formula (29) by removing the protecting groups of the 3'- and 5'-hydroxyl groups of the ribonucleic acid derivative (25) produced by Step c or Step e. 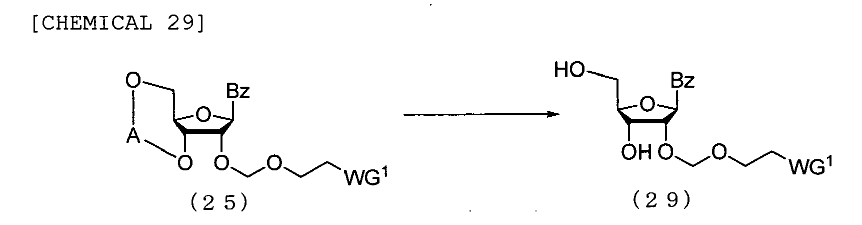 In the general formulae (25) and (29), A, B_{Z} and WG¹ have the same meanings as above.
The step can be performed by dissolving the ribonucleic acid derivative (25) in an organic solvent, and reacting a fluorinating agent alone or a mixture of a fluorinating agent and an acid (e.g., acetic acid, hydrochloric acid and sulfuric acid) mixed in an arbitrary ratio. Examples of the "fluorinating agent" to be used in the step may include ammonium fluoride, tetra n-butylammonium fluoride (TBAF), triethylamine trihydrofluoride and hydrogen fluoride pyridine. The amount of the fluorinating agent to be used may be in the range of 0.1 to 20 mol per mol, and is preferably in the range of 0.2 to 10 mol per mol of the ribonucleic acid derivative (25). The reaction temperature is preferably in the range of 0°C to 120°C. The reaction time depends on the kind of raw materials and the reaction temperature, and is preferably between 30 minutes and 24 hours. The ratio of the fluorinating agent and the acid in the mixed reagent may be in the range of 1:0.1 to 1:2, and preferably 1:1 to 1:1.2.

### (7) Step g:

Process for producing a ribonucleic acid derivative (17) by introducing a protecting group (R¹), which can be removed under acidic conditions, at the 5'-hydroxyl position of the ribonucleic acid derivative (29) produced by Step f. 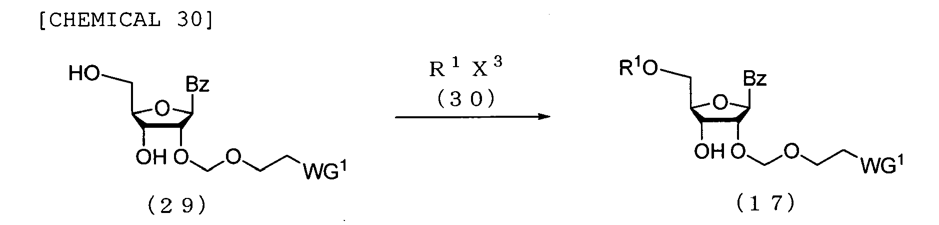 In the general formulae (17), (29) and (30), A, B_{Z}, R¹ and WG¹ have the same meanings as above. X³ represents halogen.
Examples of the "halogen" related to the X³ may include the same ones as those illustrated for the "halogen" related to the above-mentioned modified form of B_{X}.
The step can be performed by reacting R¹X³ (30) with a ribonucleic acid derivative (29) according to a known method. The amount of R¹X³ to be used may be in the range of 0.8 to 20 mol per mol, and is preferably in the range of 1 to 10 mol per mol of the ribonucleic acid derivative (29). The solvent to be used is not specifically limited unless it is involved in the reaction, and it may include, for example, acetonitrile and THF. Examples of the "base" may include organic bases such as pyridine, 2,6-dimethylpyridine, 2,4,6-trimethylpyridine, N-methylimidazole, triethylamine, tributylamine, N,N-diisopropylethylamine and 1,8-diazabicyclo[5.4.0]-7-undecene. The amount of the base to be used may be in the range of 0.8 to 20 mol per mol, and is preferably in the range of 1 to 10 mol per mol of the ribonucleic acid derivative (29). The reaction temperature is preferably in the range of 0°C to 120°C. The reaction time depends on the kind of raw materials and the reaction temperature, and is preferably between 30 minutes and 24 hours.

### (8) Step h:

Process for producing the phosphoramidite compound (B), wherein 3'-hydroxyl group is phosphoramidited, by allowing a phosphoramiditing reagent and an activating agent, if necessary, to act on a ribonucleic acid derivative (17) produced by Step b or Step f. 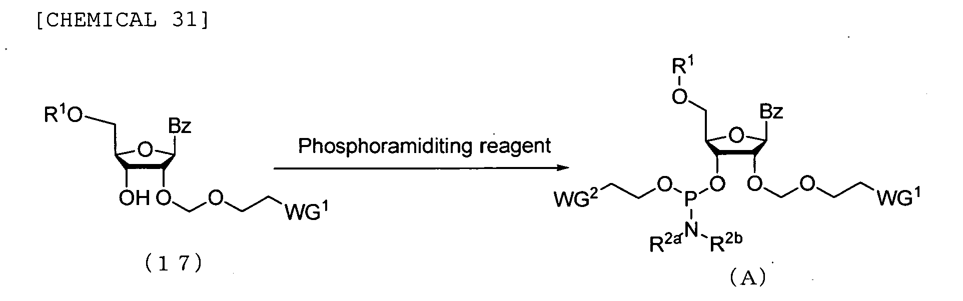 In the general formulae (17) and (B), B_{Z}, R¹, R^{2a}, R^{2b}, WG¹ and WG² have the same meanings as above.
Examples of the "phosphoramiditing reagent" may include a compound represented by the following general formula (31a) or (31b). 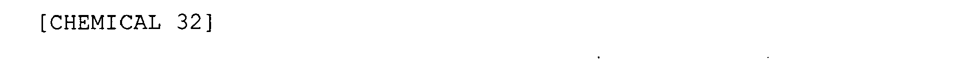 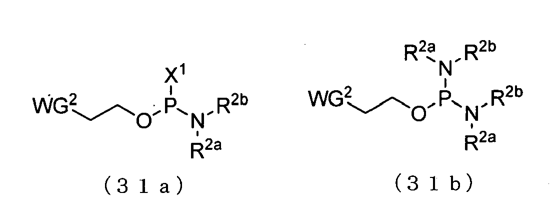 In the general formulae (31a) and (31b), R^{2a}, R^{2b} and WG² have the same meanings as above. X¹ represents halogen.
Examples of the "halogen" related to the X¹ may include the same ones as illustrated for the "halogen" related to the above-mentioned modified form of B_{X}.
The step is a reaction for phosphoramiditing the 3'-hydroxyl group by reacting the phosphoramiditing reagent with a ribonucleic acid derivative (17), and it can be performed according to a known method. An activating agent can be used if necessary. The solvent to be used is not specifically limited unless it is involved in the reaction, and it may include, for example, acetonitrile and THF. The amount of the phosphoramiditing reagent to be used may be in the range of 0.8 to 20 mol per mol, and is preferably in the range of 1 to 10 mol per mol of the ribonucleic acid derivative (17). Examples of the "activating agent" may include 1H-tetrazole, 5-ethylthiotetrazole, 5-benzylmercapto-1H-tetrazole, 4,5-dichloroimidazole, 4,5-dicyanoimidazole, benzotriazole triflate, imidazole triflate, pyridinium triflate, N,N-diisopropylethylamine and 2,4,6- collidine/N-methylimidazole. The amount of the activating agent to be used may be in the range of 0.8 to 20 mol per mol, and is preferably in the range of 1 to 10 mol per mol of the ribonucleic acid derivative (17). The reaction temperature is preferably in the range of 0°C to 120°C. The reaction time depends on the kind of raw materials and the reaction temperature, and is preferably between 30 minutes and 24 hours.
The phosphoramidite compound (B) thus produced can be isolated and purified by a method known per se, such as concentration, liquid-phase conversion, partition, solvent extraction, crystallization, recrystallization, fractional distillation or chromatography.

### EXAMPLES

The present invention will now be described in more detail with reference to Examples, to which, however, the present invention is not limited.

### Reference Example 1

### Chloromethyl 2-cyanoethyl ether

### Step 1

### Production of methyl thiomethyl 2-cyanoethyl ether

3-Hydroxypropionitrile (32 g, 450 mmol) was dissolved in 450 mL of dimethylsulfoxide, 324 mL of acetic anhydride and 231 mL of acetic acid were added thereto, and the reaction solution was stirred at room temperature for 24 hours. Sodium bicarbonate (990 g) was dissolved in 4.5 L of water, the reaction solution was added to the aqueous sodium bicarbonate solution dropwise over a period of 1 hour, and after stirring for 1 hour the mixture was subjected to extraction with ethyl acetate, the extract was dried over anhydrous magnesium sulfate, and the solvent was distilled off. The oily product obtained was purified by silica gel column chromatography to give 41 g of methylthiomethyl 2-cyanoethyl ether as a colorless oily product (yield 70%).
¹H-NMR (CDCl₃): 2.18 (s, 3H), 2.66 (t, 2H, J = 6.3 Hz), 3.77 (t, 2H, J = 6.3 Hz), 4.69 (s, 2H)

### Step 2

### Production of chloromethyl 2-cyanoethyl ether

Methylthiomethyl 2-cyanoethyl ether (3.3 g, 25 mmol) was dissolved in 70 mL of methylene chloride, 2 mL of sulfuryl chloride (25 mmol) was added dropwise, and the reaction was performed at room temperature for 1 hour. After the completion of the reaction, the solvent was distilled off under reduced pressure to give 2.5 g of the desired compound as a colorless oily product (yield 85%).
Boiling point: 84°C - 85°C (0.3 Torr)
¹H-NMR (CDCl₃): 2.72 (t, 2H, J = 6.3 Hz), 3.92 (t, 2H, J = 6.3 Hz), 5.52 (s, 2H)

### Reference Example 2

### 5'-O-(4,4'-dimethoxytrityl)-2'-O-(2-cyanoethoxymethyl)uridine 3'-O-(2-cyanoethyl N,N-diisopropylphosphoramidite)

### Step 1

### Production of 5'-O-(4,4'-dimethoxytrityl)-2'-O-(2-cyanoethoxymethyl)uridine

5'-O-(4,4'-Dimethoxytrityl)uridine (546 mg, 1 mmol) was dissolved in 4 mL of 1,2-dichloroethane, 452 mg of diisopropylethylamine (3.5 mmol) was added thereto, and 365 mg of dibutylstannyl dichloride (1.2 mmol) was further added thereto. The reaction was performed at room temperature for 1 hour. Subsequently, the reaction was performed at 80°C, 155.4 mg of chloromethyl 2-cyanoethyl ether (1.3 mmol) was added dropwise, and the reaction solution was stirred for 30 minutes. After the completion of the reaction, the reaction solution was added to an aqueous saturated sodium bicarbonate solution and subjected to extraction with methylene chloride, after which the extract was dried over anhydrous magnesium sulfate, and the solvent was distilled off. The mixture obtained was purified by chromatography on a 30-g silica gel column to give 5'-O-(4,4'-dimethoxytrityl)-2'-O-(2-cyanoethoxymethyl)uridine (197 mg, yield 34%).
¹H-NMR (CDCl₃): 2.47 (d, 1H, J = 7.8 Hz), 2.69 (t, 2H, J = 6.3 Hz), 3.55 (dd, 1H, J = 11.3, 2.2 Hz), 3.62 (dd, 1H, J = 11.3, 2.2 Hz), 3.83 (s, 6H), 3.87 (t, 2H, J = 6.3 Hz), 4.07-4.08 (m, 1H), 4.32 (dd, 1H, J = 5.3, 1.9 Hz), 4.54 (q, 1H, J = 5.3 Hz), 4.94,5.11 (2d, 2H, J=6.9Hz), 5.32 (d, 1H, J=8.2Hz), 6.00 (d, 1H, J=1.9Hz), 6.85-6.88 (m, 4H), 7.29-7.41 (m, 9H), 8.02 (d, 1H, J = 8.2 Hz), 8.53 (br.s, 1H)
ESI-Mass: 652[M+Na]⁺

### Step 2

### Production of 5'-O-(4,4'-dimethoxytrityl)-2'-O-(2-cyanoethoxymethyl)uridine 3'-O-(2-cyanoethyl N,N-diisopropylphosphoramidite)

The 5'-O-(4,4'-dimethoxytrityl)-2'-O-(2-cyanoethoxymethyl)uridine (209 mg, 0.332 mmol) obtained in Step 1 was dissolved in 2 mL of acetonitrile and 23 mg of tetrazole. (0.332 mmol), 150 mg of 2-cyanoethyl N,N,N',N'-tetraisopropylphosphorodiamidite (0.498 mmol) was added dropwise, and the reaction was performed at 45°C for 1.5 hours. After the completion of the reaction, the reaction solution was mixed with an aqueous saturated sodium bicarbonate solution and subjected to extraction with ethyl acetate, after which the extract was dried over anhydrous magnesium sulfate, and the solvent was distilled off. The mixture obtained was purified by chromatography on a 20-g silica gel column to the desired compound (200 mg, yield 73%).
ESI-Mass: 852[M+Na]⁺

### Reference Example 3

### 2'-O-(2-cyanoethoxymethyl)uridine

### Step 1

### Production of 3',5'-O-(tetraisopropyldisiloxane-1,3-diyl)-2'-O-(2-cyanoethoxym ethyl)uridine

3',5'-O-(tetraisopropyldisiloxane-1,3-diyl)uridine (150 mg, 0.3 mmol) was dissolved in 7 mL of THF under an argon atmosphere, and 54 mg of methylthiomethyl 2-cyanoethyl ether (0.4 mmol) and 100 mg of molecular sieves 4A were added, and the reaction solution was stirred for 10 minutes. The reaction was performed at 0°C, and 2 mL of a solution of trifluoromethanesulfonic acid (10 mg, 0.06 mmol) in THF was added. Then, 92 mg of N-iodosuccinimide (0.4 mmol) was added, and the reaction solution was stirred for 1 hour. After the completion of the reaction, the reaction solution was filtered through a celite pad and washed with methylene chloride, and the organic layer obtained was washed with 1 M aqueous sodium hydrogen thiosulfate solution. The organic layer was washed with aqueous saturated sodium bicarbonate solution, and dried over anhydrous magnesium sulfate, and the solvent was distilled off. The residue obtained was purified by thin-layer chromatography to give 3',5'-O-(tetraisopropyldisiloxan-1,3-diyl)-2'-O-(2-cyanoethoxymethyl)uridine (150 mg, yield 85%).
¹H-NMR (CDCl₃): 0.97-1.12 (m, 28H), 2.68-2.73 (m, 2H), 3.78-3.86 (m, 1H), 3.96-4.05 (m, 2H), 4.12-4.30 (m, 4H), 5.0-5.04 (m, 2H), 5.70 (d, 1H, J = 8.2 Hz), 5.75 (s, 1H), 7.90 (d, 1H, J = 8.2 Hz), 9.62 (br.s, 1H)
ESI-Mass: 570[M+H]⁺

### Step 2

### Production of 2'-O-(2-cyanoethoxymethyl)uridine

The 3',5'-O-(tetraisopropyldisiloxan-1,3-diyl)-2'-O-(2-cyanoethoxyme thyl)uridine (200 mg, 0.35 mmol) obtained in step 1 was dissolved in 2 mL of methanol, 65 mg of ammonium fluoride (1.76 mmol) was added thereto, and the reaction solution was stirred with heating at 50°C for 5 hours. After air-cooling, acetonitrile was added to the reaction solution. The solution was stirred, and was filtered and concentrated. The residue obtained was purified by silica gel column chromatography to give the desired compound (108 mg, yield 94%).
¹H-NMR (CD₃OD): 2.72-2.76 (t, 2H, J = 6.2 Hz), 3.68-3.92 (m, 4H), 4.00-4.03 (m, 1H), 4.26-4.32 (m, 2H), 4.81-4.95 (m, 2H), 5.71 (d, 1H, J = 8.1 Hz), 6.00 (d, 1H, J = 3.3 Hz), 8.10 (d, 1H, J = 8.1 Hz) ESI-Mass: 350[M+Na]⁺

### Reference Example 4

### Production of 5'-O-(4,4'-dimethoxytrityl)-2'-O-(2-cyanoethoxymethyl)uridine

2'-O-(2-Cyanoethoxymethyl)uridine (14 g, 43 mmol) was subjected to azeotropic distillation with pyridine, and then was dried with a vacuum pump for 30 minutes. The residue was dissolved in 300 mL of THF, 68 g of pyridine (856 mmol) and 20 g of molecular sieves 4A was added under an argon atmosphere, and the mixture was stirred for 10 minutes. To the solution was added 19.6 g of 4,4'-dimethoxytritylchloride (57.8 mmol) in three portions at a rate of one portion per hour, and the mixture was further stirred for 1 hour. After 10 mL of methanol was added and the reaction solution was stirred for 2 minutes, the reaction solution was filtered through a celite pad, and was washed with ethyl acetate. After concentrating the filtrate, the residue was dissolved in ethyl acetate, and was washed with a saturated aqueous sodium bicarbonate solution. After the organic layer was washed with saturated brine and dried over anhydrous magnesium sulfate, the solvent was distilled off. The residue obtained was purified by silica gel chromatography to give the desired compound (26.5 g, yield 98%).

### Reference Example 5

### N⁴-acetyl-5'-O-(4,4'-dimethoxytrityl)-2'-O-(2-cyanoethoxymethyl) cytidine 3'-O-(2-cyanoethyl N,N-diisopropylphosphoramidite)

### Step 1

### Production of N⁴-acetyl-5'-O-(4,4'-dimethoxytrityl)-2'-O-(2-cyanoethoxymethyl) Cytidine

N⁴-Acetyl-5'-O-(4,4'-dimethoxytrityl)cytidine (588 mg, 1 mmol) was dissolved in 4 mL of 1,2-dichloroethane, 452 mg of diisopropylethylamine (3.5 mmol) was added thereto, and then 365 mg of dibutylstannyl dichloride (1.2 mmol) was further added. The reaction was performed at room temperature for 1 hour. Then, the reaction mixture was heated to 80°C, 155.4 mg of chloromethyl 2-cyanoethyl ether (1.3 mmol) was added dropwise, and the solution was stirred for 60 minutes at 80°C. After the completion of the reaction, the reaction solution was added to an aqueous saturated sodium bicarbonate solution, and was extracted with methylene chloride. The extract was dried over anhydrous magnesium sulfate, and the solvent was distilled off. The mixture obtained was purified by chromatography on a 30-g silica gel column to give N⁴-acetyl-5'-O-(4,4'-dimethoxytrityl)-2'-O-(2-cyanoethoxymethyl) cytidine (219 mg, yield 35%).
¹H-NMR (CDCl₃): 2.19 (s, 3H), 2.56 (d, 1H, J = 8.8 Hz), 2.65 (t, 2H, J = 6.2 Hz), 3.55 (dd, 1H, J = 10.5, 2.5 Hz), 3.63 (dd, 1H, J = 10.5, 2.5 Hz), 3.82 (s, 6H), 3.86 (t, 2H, J = 6.2 Hz), 4.09-4.14 (m, 1H), 4.28 (d, 1H, J = 5.1 Hz), 4.44-4.49 (m, 1H), 4.97,5.24 (2d, 2H, J = 6.9 Hz), 5.96 (s, 1H), 6.86-6.88 (m, 4H), 7.09 (d, 1H, J = 6.9 Hz), 7.26-7.42 (m, 9H), 8.48 (d, 1H, J = 6.9 Hz), 8.59 (br.s, 1H)
ESI-Mass: 693[M+Na]⁺

### Step 2

### Production of N⁴-acetyl-5'-O-(4,4'-dimethoxytrityl)-2'-O-(2-cyanoethoxymethyl) cytidine 3'-O-(2-cyanoethyl N,N-diisopropylphosphoramidite)

The N⁴-acetyl-5'-O-(4,4'-dimethoxytrityl)-2'-O-(2-cyanoethoxymethyl) cytidine (205 mg, 0.306 mmol) obtained in Step 1 was dissolved in 2 mL of methylene chloride, 105 mg of diisopropylethylamine (0.812 mmol) was added, and 116 mg of 2-cyanoethyl N,N-diisopropyl chlorophosphoramidite (0.49 mmol) was added dropwise. The reaction solution was reacted at room temperature for 1 hour. After the completion of the reaction, the solvent was distilled off and the mixture obtained was purified by chromatography on a 20-g silica gel column to give the desired compound (242 mg, yield 91%).
ESI-Mass: 871[M+H]⁺

### Reference Example 6

### N⁴-acetyl-2'-O-(2-cyanoethoxymethyl)cytidine

### Step 1

### Production of N⁴-acetyl-3',5'-O-(tetraisopropyldisiloxane-1,3-diyl)-2'-O-(2-cy anoethoxymethyl)cytidine

N⁴-Acetyl-3',5'-O-(tetraisopropyldisiloxane-1,3-diyl)cytid ine (1.00 g, 1.89 mmol) and 500 mg of methylthiomethyl 2-cyanoethyl ether (3.79 mmol) were mixed, and the mixture was dissolved in a mixed solvent of 10 mL of toluene and 10 mL of THF. Subsequently, 975 mg of silver trifluoromethanesulfonate (3.79 mmol) was added and the solution was dried by adding molecular sieves 4A. Under ice cooling, 370 mg of N-bromosuccinimide (2.08 mmol) was added, and the solution was stirred for 10 minutes in the reaction vessel shielded from light. A further 70 mg of N-bromosuccinimide (0.39 mmol) was added and the reaction mixture was stirred for 25 minutes.
After the completion of the reaction, the reaction solution was diluted with methylene chloride, and was washed with an aqueous saturated sodium bicarbonate solution. The extract was dried over anhydrous sodium sulfate, and the solvent was distilled off. The mixture obtained was purified by silica gel column chromatography to give N⁴-acetyl-3',5'-O-(tetraisopropyldisiloxan-1,3-diyl)-2'-O-(2-cya noethoxymethyl)cytidine (936 mg, yield 81%).
¹H-NMR (CDCl₃): 0.90-1.11 (m, 28H), 2.28 (s, 3H), 2.62-2.79 (m, 2H), 3.78-3.89 (m, 1H), 3.96-4.04 (m, 2H), 4.19-4.23 (m, 3H), 4.30 (d, 1H, J = 13.6 Hz), 5.00 (d, 1H, J = 6.8 Hz), 5.09 (d, 1H, J = 6.8 Hz), 5.77 (s, 1H), 7.44 (d, 1H, J = 7.5 Hz), 8.30 (d, 1H, J = 7.5 Hz), 10.13 (s, 1H)
ESI-Mass: 611[M+H]⁺

### Step 2

### Production of N⁴-acetyl-2'-O-(2-cyanoethoxymethyl)cytidine

The N⁴-acetyl-3',5'-O-(tetraisopropyldisiloxane-1,3-diyl)-2'-O-(2-cy anoethoxymethyl)cytidine (500 mg, 0.819 mmol)obtained in step 1 was dissolved in a mixed solvent of 2.5 mL of THF and 2.5 mL of methanol, 150 mg of ammonium fluoride (4.10 mmol) was added, and then the reaction solution was reacted at 50°C for 4 hours. After the completion of the reaction, the reaction solution was diluted with acetonitrile and filtered, and the solvent was distilled off. The mixture obtained was purified by silica gel column chromatography to give the desired compound (210 mg, yield 70%).
¹H-NMR (D₂O): 2.13 (s, 3H), 2.66-2.71 (m, 2H), 3.72-3.78 (m, 3H), 3.90 (dd, 1H, J = 13.0, 2.6 Hz), 4.06-4.11 (m, 1H), 4.20 (dd, 1H, J = 7.1, 5.2 Hz), 4.29 (dd, 1H, J = 5.1, 2.9 Hz), 4.83 (d, 1H, J = 7.2 Hz), 4.94 (d, 1H, J = 7.2 Hz), 5.95 (d, 1H, J = 2.9 Hz), 7.25 (d, 1H, J = 7.6 Hz), 8.25 (d, 1H, J = 7.6 Hz)
ESI-Mass: 391[M+Na]⁺

### Reference Example 7

### Production of N⁴-acetyl-5'-O-(4,4'-dimethoxytrityl)-2'-O-(2-cyanoethoxymethyl) cytidine

2'-O-(2-Cyanoethoxymethyl)cytidine (9.9 g, 26.8 mmol) was subjected to azeotropic distillation with pyridine, and then was dried with a vacuum pump for 30 minutes. The residue was dissolved in 190 mL of THF, 43 g of pyridine (538 mmol) and 20 g of molecular sieves 4A were added under an argon atmosphere, and the mixture was stirred for 10 minutes. To the reaction solution was added 11.8 g of 4,4'-dimethoxytrityl chloride (34.9 mmol) in three portions at a rate of one portion per hour, and the mixture was stirred for a further hour. After 2 mL of methanol was added and the reaction solution was stirred for 2 minutes, the reaction solution was filtered through a celite pad, and was washed with ethyl acetate. After concentrating the filtrate by evaporation, the residue was dissolved in ethyl acetate, and was partitioned with a saturated aqueous sodium bicarbonate solution. After the organic layer was washed with saturated brine and dried over anhydrous magnesium sulfate, the solvent was distilled off.
The residue obtained was purified by silica gel chromatography to give the desired compound (15 g, yield 83%).

### Reference Example 8

### N²-acetyl-5'-O-(4,4'-dimethoxytrityl)-2'-O-(2-cyanoethoxymethyl) guanosine 3'-O-(2-cyanoethyl N,N-diisopropylphosphoramidite)

### Step 1

### Production of N²-acetyl-5'-O-(4,4'-dimethoxytrityl)-2'-O-(2-cyanoethoxymethyl) guanosine

N²-Acetyl-5'-O-(4,4'-dimethoxytrityl)guanosine (627 mg, 1 mmol) was dissolved in 4 mL of 1,2-dichloroethane, 452 mg of diisopropylethylamine (3.5 mmol) was added, and then 365 mg of dibutylstannyl dichloride (1.2 mmol) was added. And then, the reaction solution was reacted at room temperature for 1 hour. Then, the reaction mixture was heated to 80°C, 155.4 mg of chloromethyl 2-cyanoethyl ether (1.3 mmol) was added dropwise, and the solution was stirred for 60 minutes at 80°C. After the completion of the reaction, the reaction solution was mixed with an aqueous saturated sodium bicarbonate solution, and was subjected to extraction with methylene chloride. The extract was dried over anhydrous magnesium sulfate, and the solvent was distilled off. The mixture obtained was purified by chromatography on a 30-g silica gel column to give N²-acetyl-5'-O-(4,4'-dimethoxytrityl)-2'-O-(2-cyanoethoxymethyl) guanosine (450 mg, yield 63%).
¹H-NMR (CDCl₃): 1.92 (s, 3H), 2.47-2.51 (m, 2H), 2.68 (br.s, 1H), 3.30 (dd, 1H, J = 10.7, 3.8 Hz), 3.47 (dd, 1H, J = 10.7,3.8 Hz), 3.55-3.60 (m, 1H), 3.65-3.70 (m, 1H), 3.74,3.75 (2s, 6H), 4.22-4.23 (m, 1H), 4.55-4.58 (m, 1H), 4.78,4.83 (2d, 2H, J = 7.0 Hz), 5.01 (t, 1H, J = 5.1 Hz), 5.99 (d, 1H, J = 5.1 Hz), 6.76-6.79 (m, 4H), 7.17-7.44 (m, 9H), 7.88 (s, 1H), 8.36 (br.s, 1H), 12.06 (br.s, 1H)

### Step 2

### Production of N²-acetyl-5'-O-(4,4'-dimethoxytrityl)-2'-O-(2-cyanoethoxymethyl) guanosine 3'-O-(2-cyanoethyl N,N-diisopropylphosphoramidite)

The N²-acetyl-5'-O-(4,4'-dimethoxytrityl)-2'-O-(2-cyanoethoxymethyl) guanosine (400 mg, 0.563 mmol) obtained in Step 1 was dissolved in 2 mL of methylene chloride, 181 mg of diisopropylethylamine (1.4 mmol) was added, after which 161 mg of 2-cyanoethyl N,N-diisopropylchloro phosphoramidite (0.68 mmol) was added dropwise. Then, the reaction was performed at room temperature for 1 hour. After the completion of the reaction, the solvent was distilled off and the mixture obtained was purified by chromatography on a 20-g silica gel column to give the desired compound (471 mg, yield 92%).

### Reference Example 9

### N⁶-acetyl-5'-O-(4,4'-dimethoxytrityl)-2'-O-(2-cyanoethoxymethyl) adenosine 3'-O-(2-cyanoethyl N,N-diisopropylphosphoramidite)

### Step 1

### Production of N⁶-acetyl-5'-O-(4,4'-dimethoxytrityl)-2'-O-(2-cyanoethoxymethyl) adenosine

N⁶-Acetyl-5'-O-(4,4'-dimethoxytrityl)adenosine (22.0 g, 36.0 mmol) was dissolved in 170 mL of 1,2-dichloroethane, 16.3 g of diisopropylethylamine (126 mmol) was added, after which 12.1 g of dibutylstannyl dichloride (39.7 mmol) was added. Then, the reaction was performed at room temperature for 1 hour. Then, the reaction solution was heated to 80°C, stirred for 15 minutes, 4.30 g of chloromethyl 2-cyanoethyl ether (36.0 mmol) was added dropwise, and the solution was stirred for 30 minutes. After the completion of the reaction, the reaction solution was added to an aqueous saturated sodium bicarbonate solution, and was subjected to extraction with methylene chloride. The extract was dried over anhydrous magnesium sulfate, and the solvent was distilled off. The mixture obtained was purified by silica gel column chromatography to give N⁶-acetyl-5'-O-(4,4'-dimethoxytrityl)-2'-O-(2-cyanoethoxymethyl) adenosine (7.47 g, yield 33%).
¹H-NMR (CDCl₃): 2.51 (t, 2H, J = 6.2 Hz), 2.58 (d, 1H, J = 5.5 Hz), 2.61 (s, 3H), 3.45 (dd, 1H, J = 10.7,4.0 Hz), 3.54 (dd, 1H, J = 10.7, 3.2 Hz), 3.62-3.79 (m, 2H), 3.79 (s, 6H), 4.25 (br.q, 1H, J = 4.6 Hz), 4.59 (q, 1H, J = 5.2 Hz), 4.87-4.94 (m, 3H), 6.23 (d, 1H, J = 4.4 Hz), 6.80-6.83 (m, 4H), 7.22-7.32 (m, 7H), 7.40-7.43 (m, 2H), 8.20 (s, 1H), 8.61 (br.s, 1H), 8.62 (s, 1H)
ESI-Mass: 695[M+H]⁺

### Step 2

### Production of N⁶-acetyl-5'-O-(4,4'-dimethoxytrityl)-2'-O-(2-cyanoethoxymethyl) adenosine 3'-O-(2-cyanoethyl N,N-diisopropylphosphoramidite)

The N⁶-acetyl-5'-O-(4,4'-dimethoxytrityl)-2'-O-(2-cyanoethoxymethyl) adenosine (10.0 g, 14.4 mmol) obtained in Step 1 was dissolved in 75 mL of methylene chloride, and 4.7 g of diisopropylethylamine (36 mmol) was added, and 4.82 g of 2-cyanoethyl N,N-diisopropylchloro phosphoramidite (20.3 mmol) was added dropwise. Then, the reaction was performed at room temperature for 1 hour. After the completion of the reaction, the solvent was distilled off and the mixture obtained, in which about 30 mL of the solvent remained, was purified by silica gel column chromatography to give the desired compound (12.0 g, yield 93%).
ESI-Mass: 895[M+H]⁺

### Reference Example 10

### N⁶-acetyl-2'-O-(2-cyanoethoxymethyl)adenosine

### Step 1

### Production of N⁶-acetyl-3',5'-O-(tetraisopropyldisiloxan-1,3-diyl)-2'-O-(2-cya noethoxymethyl)adenosine

In 8 mL of methylene chloride was suspended 245 mg of N-iodosuccinimide (1.09 mmol) and 280 mg of silver trifluoromethanesulfonate (1.09 mmol), and the solution was dried by adding molecular sieves 4A. To the reaction solution was added a solution of N⁶-acetyl-3',5'-O-(tetraisopropyldisiloxane-1,3-diyl)adenosine (400 mg, 0.73 mmol) and 145 mg of methylthiomethyl 2-cyanoethyl ether (1.11 mmol) in 4 mL of methylene chloride under ice cooling, and the reaction mixture was stirred for 3 hours. After the completion of the reaction, the reaction mixture was diluted with methylene chloride, and was washed with aqueous sodium thiosulfate solution and aqueous saturated sodium bicarbonate solution. The extract was dried over anhydrous magnesium sulfate, and the solvent was distilled off. The mixture obtained was purified by silica gel column chromatography to give N⁶-acetyl-3',5'-O-(tetraisopropyldisiloxan-1,3-diyl)-2'-O-(2-cya noethoxymethyl)adenosine (201 mg, yield 45%).
¹H-NMR (CDCl₃): 0.98-1.11 (m, 28H), 2.62 (s, 3H), 2.69 (td, 2H, 6.5, J = 1.5 Hz), 3.81-3.89 (m, 1H), 4.02-4.09 (m, 2H), 4.17 (d, 1H, J = 9.4 Hz), 4.28 (d, 1H, J = 13.4 Hz), 4.50 (d, 1H, J = 4.5 Hz), 4.67 (dd, 1H, J = 8.8,4.5 Hz), 5.02 (d, 1H, J = 7.0 Hz), 5.08 (d, 1H, J = 7.0 Hz), 6.10 (s, 1H), 8.34 (s, 1H), 8.66 (s, 1H), 8.67 (s, 1H) ESI-Mass: 636[M+H]⁺

### Step 2

### Production of N⁶-acetyl-2'-O-(2-cyanoethoxymethyl)adenosine

The N⁶-acetyl-3',5'-O-(tetraisopropyldisiloxane-1,3-diyl)-2'-O-(2-cyanoethoxymethyl)adenosine (300 mg, 0.47 mmol) obtained in Step 1 was dissolved in a mixed solvent of 0.1 mL of acetic acid and 2 mL of 0.5 M TBAF / THF solution, and the reaction solution was stirred at room temperature for 2 hours. After the completion of the reaction, the reaction mixture obtained was purified by silica gel column chromatography to give the desired compound (160 mg, yield 86%).
¹H-NMR (DMSO-d₆): 2.25 (s, 3H), 2.53-2.68 (m, 2H), 3.41-3.46 (m, 1H), 3.56-3.64 (m, 2H), 3.69-3.73 (m, 1H), 4.00-4.01 (m, 1H), 4.36-4.37 (m, 1H), 4.72-4.78 (m, 3H), 5.20 (bt, 2H), 5.41 (d, 1H, J = 5.2 Hz), 6.17 (d, 1H, J = 5.7 Hz), 8.66 (s, 1H), 8.72 (s, 1H), 10.72 (s, 1H)
ESI-Mass: 415[M+Na]⁺

### Reference Example 11

### Production of N⁶-acetyl-2'-O-(2-cyanoethoxymethyl)adenosine

N⁶-Acetyl-2'-O-(2-cyanoethoxymethyl)adenosine (9.50 g, 24.2 mmol) was dissolved in 100 mL of dehydrated pyridine, and then was dried by concentration. Then, the residue was dissolved in 100 mL of dehydrated pyridine under an argon atmosphere. Under ice cooling, 10.7 g of 4,4'-dimethoxytrityl chloride (31.2 mmol) was added, and the reaction was performed at room temperature for 1 hour and 20 minutes. After the completion of the reaction, the reaction solution was diluted with methylene chloride, and was washed with water. The extract was dried over anhydrous sodium sulfate, and the solvent was distilled off. The mixture obtained was purified by silica gel column chromatography to give the desired compound (13.8 g, yield 82%).

### Reference Example 12

### N²-Phenoxyacetyl-5'-O-(4,4'-dimethoxytrityl)-2'-O-(2-cyanoethoxy methyl)guanosine N,N-diisopropylphosphoramidite)

### Step 1

### Production of N²-phenoxyacetyl-5'-O-(4,4'-dimethoxytrityl)-2'-O-(2-cyanoethoxy methyl)guanosine

N²-Phenoxyacetyl-5'-O-(4,4'-dimethoxytrityl)guanosine (720 mg, 1 mmol) was dissolved in 4 mL of 1,2-dichloroethane, 452 mg of diisopropylethylamine (3.5 mmol) was added, after which 365 mg of dibutylstannyl dichloride (1.2 mmol) was added. Then, the reaction was performed at room temperature for 1 hour. Then, the reaction was performed at 80°C, and 155.4 mg of chloromethyl 2-cyanoethyl ether (1.3 mmol) was added dropwise, and the solution was stirred for 60 minutes. After the completion of the reaction, the reaction solution was mixed with an aqueous saturated sodium bicarbonate solution, and was subjected to extraction with methylene chloride. The extract was dried over anhydrous magnesium sulfate, and the solvent was distilled off. The mixture obtained was purified by chromatography on a 30-g silica gel column to give N²-phenoxyacetyl-5'-O-(4,4'-dimethoxytrityl)-2'-O-(2-cyanoethoxy methyl)guanosine (384 mg, yield 48%).
¹H-NMR (CDCl₃): 2.47-2.51 (m, 2H), 2.58 (br.s, 1H), 3.42 (dd, 1H, J = 10.1, 3.8 Hz), 3.46 (dd, 1H, J = 10.1, 3.8 Hz), 3.53-3.57 (m, 1H), 3.69-3.73 (m, 1H), 3.77 (s, 6H), 4.24-4.26 (m, 1H), 4.48-4.50 (m, 1H), 4.61-4.65 (m, 2H), 4.83,4.87 (2d, 2H, J = 7.0 Hz), 4.88 (t, 1H, J = 5.7 Hz), 6.05 (d, 1H, J = 5.7 Hz), 6.80-6.82 (m, 4H), 6.92-6.96 (m, 3H), 7.07-7.11 (m, 2H), 7.20-7.42 (m, 9H), 7.84 (s, 1H), 8.99 (s, 1H), 11.81 (br.s, 1H)
ESI-Mass: 825[M+Na]⁺

### Step 2

### Production of N²-phenoxyacetyl-5'-O-(4,4'-dimethoxytrityl)-2'-O-(2-cyanoethoxymethyl)guanosine 3'-O-(2-cyanoethyl N,N-diisopropylphosphoramidite)

The N²-phenoxyacetyl-5'-O-(4,4'-dimethoxytrityl)-2'-O-(2-cyanoethoxy methyl) guanosine (320 mg, 0.399 mmol) obtained in Step 1 was dissolved in 4 mL of methylene chloride, 128.8 mg of diisopropylethylamine (0.996 mmol) was added, and 141.5 mg of 2-cyanoethyl N,N-diisopropylchlorophosphoramidite (0.598 mmol) was added dropwise. Then, the reaction was performed at room temperature for 1 hour. After the completion of the reaction, the solvent was distilled off and the mixture obtained was purified by chromatography on a 30-g silica gel column to give the desired compound (316 mg, yield 79%).
ESI-Mass: 1,003[M+H]⁺

### Reference Example 13

### N²-Phenoxyacetyl-2'-O-(2-cyanoethoxymethyl)guanosine

### Step 1

### Production of N²-phenoxyacetyl-3',5'-O-(tetraisopropyldisiloxane-1,3-diyl)-2'-O-(2-cyanoethoxymethyl)guanosine

N²-Phenoxyacetyl-3',5'-O-(1,3-tetraisopropyldisiloxane-1,3 -diyl) guanosine (2.0 g, 3.0 mmol) was dissolved in THF 16 mL, 0.99 g of methylthiomethyl 2-cyanoethyl ether (7.6 mmol) and 1.0 g of molecular sieves 4A were added, and the reaction solution was stirred at -45°C for 10 minutes under an argon atmosphere. After a solution of 0.68 g of trifluoromethanesulfonic acid (4.5 mmol) in 5 mL of THF was added and the reaction solution was stirred, 1.02 g of N-iodosuccinimide (4.5 mmol) was added, and the reaction solution was stirred for 15 minutes. After saturated aqueous sodium bicarbonate solution was added to the reaction solution and the reaction solution was filtered, the organic layer was washed with 1 M aqueous sodium hydrogen thiosulfate solution. The reaction solution was then washed sequentially with water and saturated brine, the extract was dried over anhydrous magnesium sulfate, and the solvent was distilled off. The residue obtained was purified by silica gel chromatography to give N²-phenoxyacetyl-3',5'-O-(tetraisopropyldisiloxan-1,3-diyl)-2'-O -(2-cyanoethoxymethyl)guanosine (2.0 g, yield 89%).
¹H-NMR (CDCl₃): 0.99-1.11 (m, 28H), 2.59-2.77 (m, 2H), 3.82-4.05 (m, 3H), 4.15 (d, 1H, J = 9.3 Hz), 4.25-4.35 (m, 2H), 4.52-4.56 (dd, 1H, J = 9.3, 4.3 Hz), 5.00,5.07 (2d, 2H, J = 7.2 Hz), 5.95 (s, 1H) 6.99-7.12 (m, 3H), 7.35-7.40 (m, 2H), 8.09 (s, 1H), 9.38 (br.s, 1H), 11.85 (br.s, 1H)
ESI-Mass: 766[M+Na]⁺

### Step 2

### Production of N²-phenoxyacetyl-2'-O-(2-cyanoethoxymethyl)guanosine

A solution consisting of 0.14 mL of acetic acid (0.14 mmol) and 2.83 mL of 1 M TBAF in THF (2.83 mmol) was prepared. The N²-phenoxyacetyl-3'5'-O-(tetraisopropyldisiloxane-1,3-diyl)-2'-O - (2-cyanoethoxymethyl) guanosine (1.0 g, 1.35 mmol) obtained in Step 1 was dissolved in 2.83 mL of THF, and the solution prepared above was added, and the reaction was performed at room temperature for 1 hour under an argon atmosphere. The reaction solution was concentrated under reduced pressure, and the residue was dissolved in methylene chloride and purified by silica gel column chromatography to give the desired compound (0.67 g, yield 99%).
¹H-NMR (DMSO-d₆): 2.59-2.66 (m, 2H), 3.41-3.63 (m, 4H), 3.98 (m, 1H), 4.32 (m, 1H), 4.58-4.62 (t, 1H, J = 5.3 Hz), 4.71-4.78 (dd, 2H, J = 13.1, 6.8 Hz), 4.87 (s, 2H), 5.12 (s, 1H) 5.37 (s, 1H), 5.97 (d, 1H, J = 6.1 Hz) 6.96-6.99 (m, 3H), 7.28-7.34 (m, 2H), 8.30 (s, 1H), 11.78 (br.s, 2H)
ESI-Mass: 500[M-H]⁻

### Reference Example 14

### Production of N²-phenoxyacetyl-5'-O-(4,4'-dimethoxytrityl)-2'-O-(2-cyanoethoxymethyl)guanosine

N²-Phenoxyacetyl-2'-O-(2-cyanoethoxymethyl)guanosine (660 mg, 1.32 mmol) was subjected to azeotropic distillation with pyridine, and then was dried with a vacuum pump for 30 minutes. The residue was dissolved in 9 mL of THF, 2.1 g of pyridine (26.4 mmol) and 600 mg of molecular sieves 4A were added under an argon atmosphere, and the reaction solution was stirred for 10 minutes. To the solution was added 540 mg of 4, 4'-dimethoxytritylchloride (1.58 mmol) in three portions at a rate of one portion per hour, and the reaction solution was stirred for a further hour. After 2 mL of methanol was added and the reaction solution was stirred for 2 minutes, the reaction solution was filtered through a celite pad, and was washed with ethyl acetate. After concentrating the filtrate by evaporation, the residue was dissolved in ethyl acetate, and was partitioned with a saturated aqueous sodium bicarbonate solution. After the organic layer was washed with saturated brine and dried over anhydrous magnesium sulfate, the solvent was distilled off. The residue obtained was purified by silica gel chromatography to give the desired compound (800 mg, yield 75%).

### Reference Example 15

### N⁶-Acetyl-3',5'-O-(tetraisopropyldisiloxane-1,3-diyl)-2'-O-(2-cy anoethoxymethyl)adenosine

### Step 1

### Production of N⁶-acetyl-3',5'-O-(tetraisopropyldisiloxane-1,3-diyl)-2'-O-methy lthiomethyl adenosine

N⁶-Acetyl-3',5'-O-(tetraisopropyldisiloxan-1,3-diyl)adenos ine (2.00 g, 3.62 mmol) was dissolved in 25 mL of dimethylsulfoxide, 17.5 mL of acetic anhydride and 12.5 mL of acetic acid were added, and the reaction solution was stirred at room temperature for 14 hours. After the completion of the reaction, the reaction solution was added to 200 mL of water, extracted with ethyl acetate, and was washed with saturated aqueous sodium bicarbonate solution. The extract was dried over anhydrous sodium sulfate, and the solvent was distilled off. The mixture obtained was subjected to purification with silica gel column chromatography to give N⁶-acetyl-3',5'-O-(tetraisopropyldisiloxan-1,3-diyl)-2'-O-methyl thiomethyl adenosine (1.36 g, yield 61%).
¹H-NMR (CDCl₃): 0.96-1.11 (m, 28H), 2.20 (s, 3H), 2.61 (s, 3H), 4.03 (dd, 1H, J = 13.4, 2.4 Hz), 4.18 (d, 1H, J = 9.1 Hz), 4.27 (d, 1H, J = 13.4 Hz), 4.63-4.71 (m, 2H), 5.00 (d, 1H, J = 11.5 Hz), 5.07 (d, 1H, J = 11.5 Hz), 6.09 (s, 1H), 8.31 (s, 1H), 8.65 (s, 1H), 8.69 (s, 1H)
ESI-Mass: 635[M+Na]⁺

### Step 2

### Production of N⁶-acetyl-3',5'-O-(tetraisopropyldisiloxane-1,3-diyl)-2'-O-(2-cy anoethoxymethyl)adenosine

The N⁶-acetyl-3',5'-O-(tetraisopropyldisiloxane-1,3-diyl)-2'-O-methy lthiomethyl adenosine (1.00 g, 1.63 mmol) obtained in Step 1 was dissolved in 25 mL of THF. To the reaction solution was added 5.88 g of 3-hydroxypropionitrile (82.7 mmol), and the solution was dried by adding molecular sieves 4A, and was cooled to -45°C. To the reaction solution was added 440 mg of N-iodosuccinimide (1.96 mmol) and then 490 mg of trifluoromethanesulfonic acid (3.26 mmol), and the reaction solution was stirred at -45°C for 15 minutes. After the completion of the reaction, the reaction solution was neutralized by adding triethylamine while cooling, and diluted with methylene chloride. The reaction solution was washed with aqueous sodium thiosulfate solution and saturated aqueous sodium bicarbonate solution, the extract was dried over anhydrous sodium sulfate, and the solvent was distilled off. The mixture obtained was subjected to purification with silica gel column chromatography to give the desired compound (722 mg, yield 71%).

### Test Example 1

### Effect of acylation reaction activator and scavenger phenoxyacetic acid derivative to be used in capping Step 1

A commercially available CPG solid support (333 mg, 15 µmol) carrying 5'-O-(4,4'-dimethoxytrityl)thymidine was placed in a column with a glass filter, and by using an automated nucleic acid synthesizer (Expedite^{™}: Applied Biosystems), the oligonucleic acid (adenylyl-[3'→5']-cytidylyl-[3'→5']-uridylyl-[3'→5']-guanylyl-[3'→5']-adenylyl-[3'→5']-cytidylyl-[3'→5']-uridylyl-[3'→5']-g uanylyl-[3'→5']-adenylyl-[3'→5']-cytidylyl-[3'→5']-uridylyl-[3 '→5']-guanylyl-[3'→5']-adenylyl-[3'→5']-cytidylyl-[3'→5']-uri dylyl-[3'→5']-guanylyl-[3'→5']-adenylyl-[3'→5']-cytidylyl-[3' →5']-uridylyl-[3'→5']-guanylyl-[3'→5']-thymidine) was synthesized.
Condensation of a nucleic acid monomer compound was carried out 20 times by using N⁴-acetyl-5'-O-(4,4'-dimethoxytrityl)-2'-O-(2-cyanoethoxymethyl) adenosine 3'-O-(2-cyanoethyl N,N-diisopropylphosphoramidite), N⁴-acetyl-5'-O-(4,4'-dimethoxytrityl)-2'-O-(2-cyanoethoxymethyl) cytidine 3'-O-(2-cyanoethyl N,N-diisopropyl phosphoramidite), 5'-O-(4,4'-dimethoxytrityl)-2'-O-(2-cyanoethoxymethyl) uridine 3'-O-(2-cyanoethyl N,N-diisopropylphosphoramidite), and N⁴-phenoxyacetyl-5'-O-(4,4'-dimethoxytrityl)-2'-O-(2-cyanoethoxy methyl)guanosine 3'-O-(2-cyanoethyl N,N-diisopropylphosphor amidite) as nucleic acid monomer compounds, benzylmercaptotetrazole as a condensation catalyst, an iodine solution as an oxidizing agent, and any one of the 4 kinds of reagent combination shown in the following Table 1 as reagents to be used in the capping step.

**[TABLE 1]**

| | Acylating agent | Acylation reaction activator | scavenger of phenoxyacetic acid derivative | Solvent |
|---|---|---|---|---|
| 1 | Phenoxyacetic anhydride | NMI | Pyridine | THF |
| | Composition of reagent: 0.1 M Phenoxyacetic anhydride in THF solution and NMI / pyridine / THF solution (Cap B solution 1 (Wako Pure Chemical Industries, Ltd.), 10 vol% NMI / pyridine - THF solution (pyridine:THF = 1:8)) | | | |
| 2 | Phenoxyacetic anhydride | 4-DMAP | Pyridine | THF |
| | Composition of reagent: 0.1 M Phenoxyacetic anhydride in THF solution and 4-DMAP / pyridine / THF solution (4-DMAP (6.5 g), pyridine (10 mL), THF (90 mL)) | | | |
| 3 | Phenoxyacetic anhydride | 2-DMAP | Pyridine | THF |
| | Composition of reagent: 0.1 M Phenoxyacetic anhydride in THF solution and 2-DMAP / pyridine / THF solution (2-DMAP (6.5 g), pyridine (10 mL), THF (90 mL)) | | | |
| 4 | Phenoxyacetic anhydride | 2-DMAP | 2,6-lutidine | THF |
| | Composition of reagent: 0.1 M Phenoxyacetic anhydride in THF solution and 2-DMAP / 2,6-lutidine / THF solution (2-DMAP (6.5 g), 2,6-lutidine (10 mL), THF (90 mL)) | | | |

Each oligonucleic acid produced under the above-mentioned conditions was cleaved from the CPG solid support, and the protecting groups at every phosphate site and every base site were removed at 40°C for 4 hours by using a mixture of aqueous ammonia and ethanol (3:1) as the cleaving agent. Each reaction mixture obtained was filtered, and the resulting filtrate was concentrated under reduced pressure. Then, the protecting group at the 2'-hydroxyl position was removed at room temperature for 3 hours by using 1 M TBAF in THF solution containing 1% nitromethane. A portion of each of the crude oligonucleic acids produced under the above-mentioned conditions was removed and subjected to HPLC analysis.

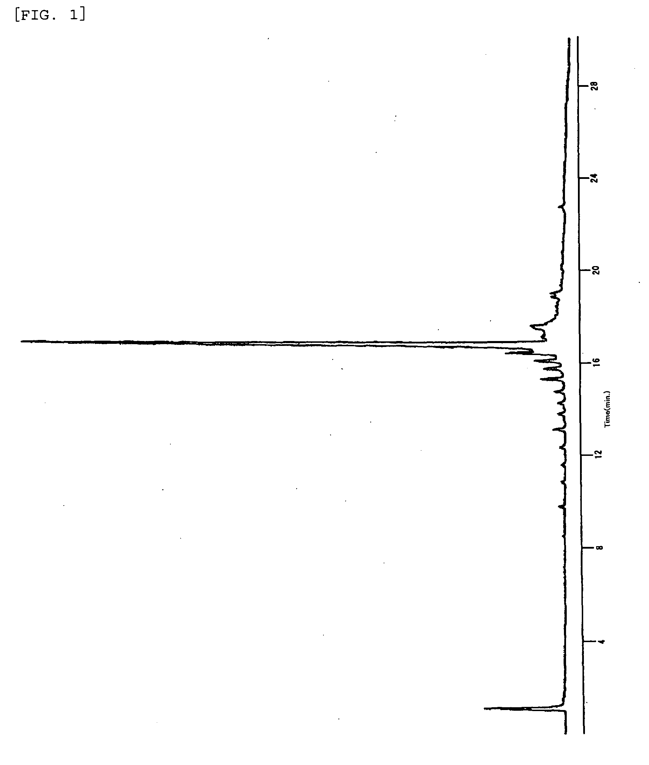

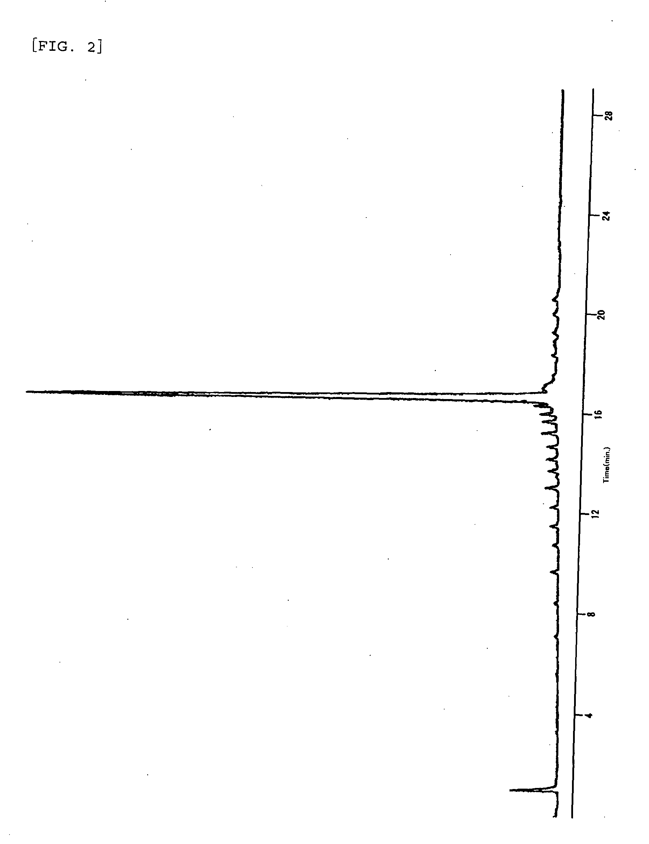

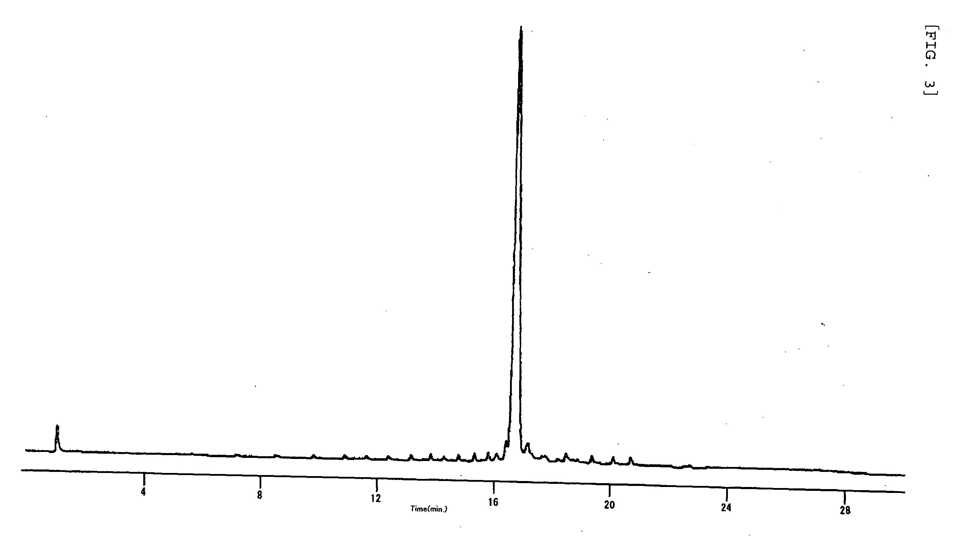

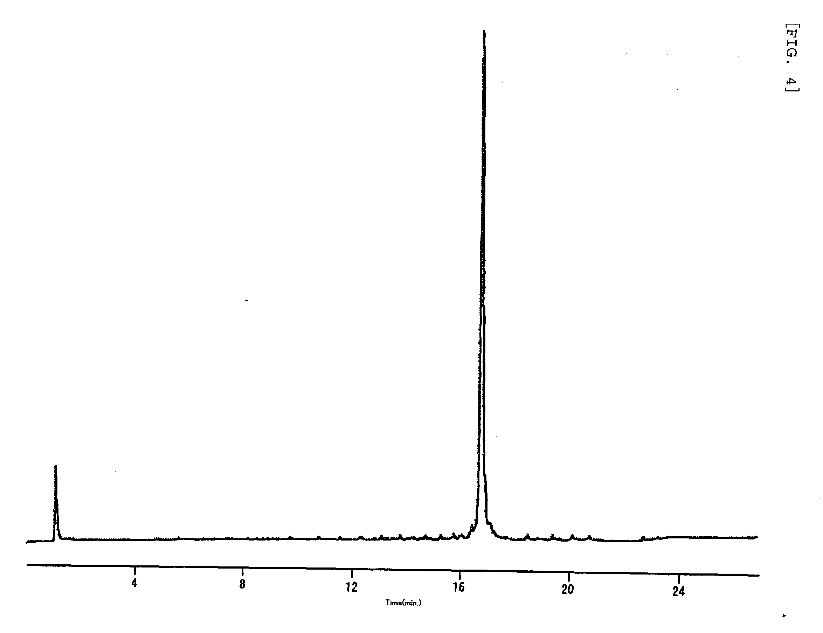

Figs. 1, 2, 3 and 4 show the results of HPLC analysis under the following measurement conditions of crude oligonucleic acids produced by using the above-mentioned reagents 1, 2, 3 and 4, respectively.

### Measurement conditions:

### HPLC apparatus

Liquid feed unit: LC-10AT VP (Shimadzu Corporation)
Detector: SPD-10AVP (Shimadzu Corporation)

### Anion exchange HPLC column:

DNAPac PA-100 <4 mm φ x 250 mm> (Dionex Corporation)

### Column temperature: 50°C

### Mobile phase

Gradient: Linear gradient, 20 min (Solution B: 5% - 25%)
Solution A: 25 mM Tris-HCl buffer including 10% acetonitrile
Solution B: 25 mM Tris-HCl buffer including 10% acetonitrile and 700 mM sodium perchlorate

### Flow rate of mobile phase: 1 mL/min

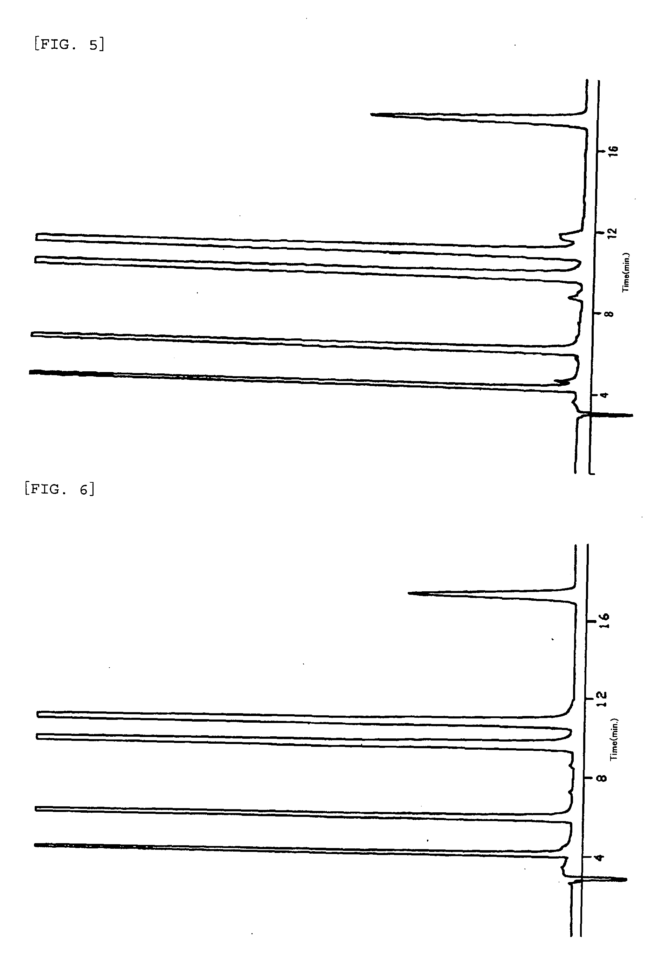

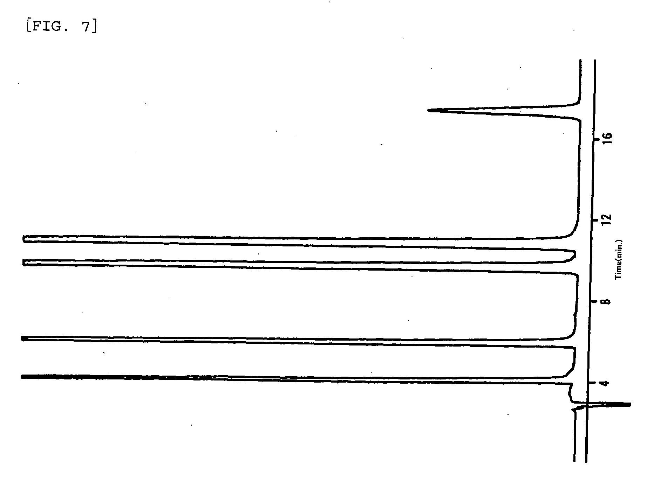

Wavelength for detection with ultraviolet-visible spectrophotometer: 260 nm
Each of the reaction mixtures was then added dropwise to ethanol, thereby causing a precipitate to form, and the precipitate was separated from the mother liquor by centrifugation. After the mother liquor was removed by decantation, the precipitate was applied to an anion exchange column (DEAE) and unwanted peaks were removed, followed by purification with an eluent (10 mM phosphate buffer containing 1.0 M aqueous sodium chloride). The resulting solution was dialyzed, whereby the desired compound was obtained. To 200 µL of the sample diluted to about 20 OD/mL with sterile water, nuclease P1 (0.5 units) was added, and the reaction was allowed to proceed at 37°C for 48 hours. Then, alkaline phosphatase (5 units) and a buffer (50 mM Tris-HCl buffer containing 1 mM MgCl₂, 0.1 mM ZnCl₂, and 1 mM spermidine, pH 9.3) were added thereto, and the reaction was allowed to proceed at 37°C for 24 hours, whereby the compound was enzymatically degraded. Each of the enzymatically degraded products was subjected to HPLC analysis under the following measurement conditions (see Figs. 5, 6 and 7).
Figs. 5, 6 and 7 show the results of HPLC analysis under the following measurement conditions of enzymatically degraded products of oligonucleic acids produced by using the above-mentioned reagents columns 2, 3 and 4, respectively.

### Measurement conditions:

### HPLC apparatus

Liquid feed unit: LC-10AS (Shimadzu Corporation)
Detector: SPD-6AV (Shimadzu Corporation)

### Reverse-phase HPLC column:

Develosil ODS-UG-5 reverse-phase column <4.6 mm φ x 250 mm> (Nomura Chemical Co., Ltd.)

### Column temperature: 40°C

### Mobile phase

Gradient: Isocratic, 20 min
50 mM aqueous potassium dihydrogen phosphate solution (pH 3.0) : methanol = 20:1

### Flow rate of mobile phase: 1 mL/min

Wavelength for detection with ultraviolet-visible spectrophotometer: 260 nm
From the results shown in Figs. 1 to 4, it is apparent that the capping efficiency was higher when 2-DMAP, rather than NMI or 4-DMAP, was used as the acylation reaction activator. It was further confirmed that the capping efficiency was higher when 2,6-lutidine rather than pyridine was used as the scavenger of the phenoxyacetic acid derivative.
From the results shown in Figs. 5 to 7, the formation of a small amount of guanosine-derived 2,6-diaminopurine was confirmed at a retention time of about 8.5 minutes, when NMI or 4-DMAP was used as the acylation reaction activator. However, when 2-DMAP was used as the acylation reaction activator instead, the formation of 2,6-diaminopurine was significantly decreased, and when in addition the base was changed from pyridine to 2,6-lutidine, the formation of 2,6-diaminopurine was not detected.

### Test Example 2

### Effect of acylation reaction activator and scavenger of phenoxyacetic acid derivative to be used in capping step 2

A commercially available CPG solid support (333 mg, 15 µmol) carrying 5'-O-(4,4'- dimethoxytrityl)thymidine was placed in a column with a glass filter, and by using an automated nucleic acid synthesizer (Expedite^{™}: Applied Biosystems), the oligonucleic acid (cytidylyl-[3'→5']-uridylyl-[3'→5']-uridylyl-[3'→5']-adenylyl-[3'→5']-cytidylyl-[3'→5']-guanylyl-[3'→5']-cytidylyl-[3'→5']-uridylyl-[3'→5']-guanylyl-[3'→5']-adenylyl-[3'→5']-guanylyl-[3 '→5']-uridylyl-[3'→5']-adenylyl-[3'→5']-cytidylyl-[3'→5']-uri dylyl-[3'→5']-uridylyl-[3'→5']-cytidylyl-[3'-5']-guanylyl-[3' →5']-adenylyl-[3'→5']-thymidyl-[3'→5']-thymidine) was synthesized.
Condensation of a nucleic acid monomer compound was carried out 20 times by using N⁴-acetyl-5'-O-(4,4'-dimethoxytrityl)-2'-O-(2-cyanoethoxymethyl) adenosine 3'-O-(2-cyanoethyl N,N-diisopropylphosphoramidite), N⁴-acetyl-5'-O-(4,4'-dimethoxytrityl)-2'-O-(2-cyanoethoxymethyl) cytidine 3'-O-(2-cyanoethyl N,N-diisopropyl phosphoramidite), 5'-O-(4,4'-dimethoxytrityl)-2'-O-(2-cyanoethoxymethyl) uridine 3'-O-(2-cyanoethyl N,N-diisopropylphosphoramidite), and N⁴-phenoxyacetyl-5'-O-(4,4'-dimethoxytrityl)-2'-O-(2-cyanoethoxy methyl)guanosine 3'-O-(2-cyanoethyl N,N-diisopropylphosphor amidite) as nucleic acid monomer compounds, benzylmercaptotetrazole as a condensation catalyst, an iodine solution as an oxidizing agent, and any one of the 3 kinds of reagent combinations shown in the following Table 2 as reagents to be used in the capping step.
Each oligo-RNA produced under the above-mentioned conditions was cleaved from the CPG solid support, and the protecting groups at every phosphate site and every base site were removed at 40°C for 4 hours by using a mixture of aqueous ammonia and ethanol (3:1) as the cleaving agent. Each reaction mixture obtained was filtered, and the resulting filtrate was concentrated under reduced pressure. Then, the protecting group at the 2'-hydroxyl position was removed at room temperature for 3 hours by using 1 M TBAF in THF solution containing 1% nitromethane. Subsequently, each of the reaction mixtures was added dropwise to ethanol thereby, causing a precipitate to form, and the precipitate was separated from the mother liquor by centrifugation. After the mother liquor was removed by decantation, the precipitate was applied to an anion exchange column (DEAE) and unwanted peaks were removed, followed by purification with an eluent (10 mM phosphate buffer containing 1.0 M aqueous sodium chloride). The resulting solution was dialyzed, whereby the desired compound was obtained. The yield amounts and yield ratios of the resulting oligonucleic acids in the case of using the above-mentioned respective reagents are shown below.

**[TABLE 2]**

| | Acylating agent | Acylation reaction activator | scavenger of phenoxyacetic acid derivative | Solvent | Yield amount (mg) | Yield ratio (%) |
|---|---|---|---|---|---|---|
| 1 | Phenoxyaceti c anhydride | NMI | Pyridine | THF | 23 | 23 |
| | Composition of reagent: 0.1 M Phenoxyacetic anhydride in THF solution and NMI / pyridine / THF solution (Cap B solution 1 (Wako Pure Chemical Industries, Ltd.), 10 vol% NMI / pyridine - THF solution (pyridine:THF = 1:8)) | | | | | |
| 2 | Phenoxyaceti c anhydride | 4-DMAP | Pyridine | THF | 33 | 33 |
| | Composition of reagent: 0.1 M Phenoxyacetic anhydride in THF solution and 4-DMAP / pyridine / THF solution (4-DMAP (6.5 g), pyridine (10 mL), THF (90 mL)) | | | | | |
| 3 | Phenoxyaceti c anhydride. | 2-DMAP | 2,6-lutidine | THF | 48 | 48 |
| | Composition anhydride in / THF solution mL), THF (90 | of reagent: THF solution (2-DMAP (6.5 mL)) | 0.1 M and 2-DMAP / 2, g), 2,6-lutidine | Phenoxyacetic 6-lutidine (10 | | |

It was confirmed by MALDI-TOF-MS that the resulting compound was the desired one. The actual values were 6607.69 [M+H]⁺, 6609.97 [M+H]⁺, and 6607.91 [M+H]⁺, respectively, while the calculated value was 6607.06 [M+H]⁺.
From the above results, it was found that the isolated yield of an oligonucleic acid is dramatically improved by using 2-DMAP as the acylation reaction activator and 2,6-lutidine as the scavenger of the phenoxyacetic acid derivative in the capping step.

### [Industrial applicability]

According to the capping step of the present invention, it is possible to produce an oligonucleic acid derivative (12) in which the 5'-hydroxyl group of a ribose of an oligonucleic acid derivative (2) is protected with an acyl group with a satisfactory efficiency. Further, according to the capping step of the present invention, 2,6-DAP is not formed as a byproduct, unlike the case where 4-DMAP is used as the acylation reaction activator.
As described above, according to the capping step of the present invention, a simple production of an oligonucleic acid with a high purity is possible.

### Brief Description of the Drawings

FIG. 1 represents a chromatogram provided by HPLC analysis. In the figure, the vertical axis indicates the time (minutes), and the horizontal axis indicates the optical absorbance.
FIG. 2 represents a chromatogram provided by HPLC analysis. In the figure, the vertical axis indicates the time (minutes), and the horizontal axis indicates the optical absorbance.
FIG. 3 represents a chromatogram provided by HPLC analysis. In the figure, the vertical axis indicates the time (minutes), and the horizontal axis indicates the optical absorbance.
FIG. 4 represents a chromatogram provided by HPLC analysis. In the figure, the vertical axis indicates the time (minutes), and the horizontal axis indicates the optical absorbance.
FIG. 5 represents a chromatogram provided by HPLC analysis. In the figure, the vertical axis indicates the time (minutes), and the horizontal axis indicates the optical absorbance.
FIG. 6 represents a chromatogram provided by HPLC analysis. In the figure, the vertical axis indicates the time (minutes), and the horizontal axis indicates the optical absorbance.
FIG. 7 represents a chromatogram provided by HPLC analysis. In the figure, the vertical axis indicates the time (minutes), and the horizontal axis indicates the optical absorbance.

## Claims

1. A method for producing an oligonucleic acid derivative represented by the following general formula (12), **characterized by** using a phenoxyacetic acid derivative anhydride represented by the following general formula (11a) as an acylating agent and a pyridine derivative represented by the following general formula (11b) or (11c) as the acylation reaction activator, in a capping step for protecting the 5'-hydroxyl group of a ribose of an oligonucleic acid derivative represented by the following general formula (2) with an acyl group. 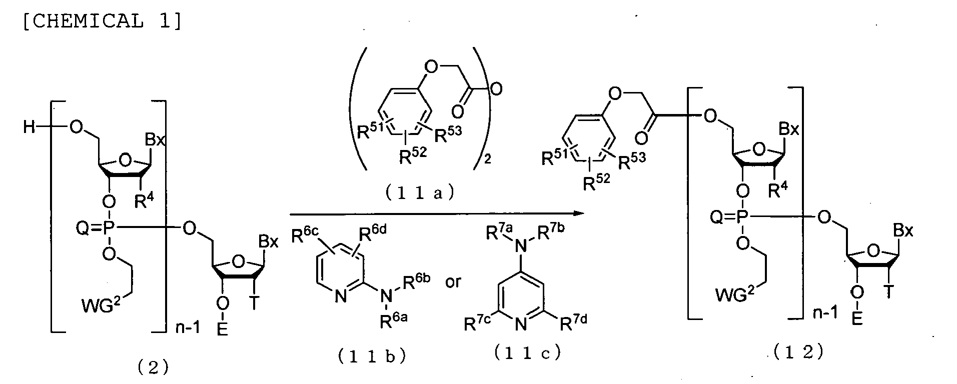 In the general formulae (2), (11a), (11b), (11c) and (12), each Bₓ independently represents a nucleobase which may have protecting groups or a modified form thereof. n represents an integer in the range of 1 to 200. Each Q independently represents O or S. Each WG² represents an electron-withdrawing group. R⁵¹, R⁵² and R⁵³ are the same or different and each represents H, alkyl or halogen. R^{6a}, R^{6b}, R^{7a}, R^{7b}, R^{7c} and R^{7d} are the same or different and each represents alkyl. R^{6c} and R^{6d} are the same or different and each represents H or alkyl. Each R⁴ independently represents H, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy or a substituent represented by the following general formula (3). 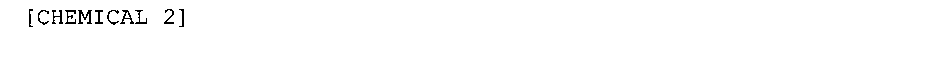 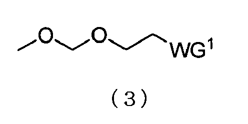 In the general formula (3), WG¹ represents an electron-withdrawing group. E represents acyl or a substituent represented by the following general formula (4). 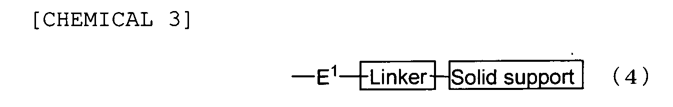 In the general formula (4), E¹ represents a single bond or a substituent represented by the following general formula (5). 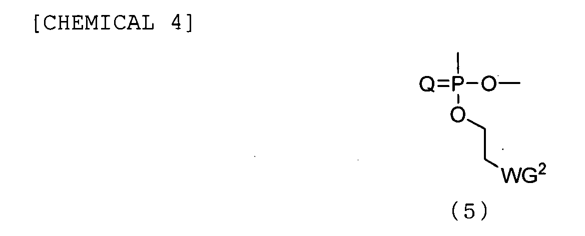 In the general formula (5), Q and WG² have the same meanings as above. T represents H, acyloxy, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy, a substituent represented by the above general formula (3) or a substituent represented by the above general formula (4), with the proviso that either E or T is a substituent (4).

2. The method for producing the oligonucleic acid derivative according to claim 1, wherein the pyridine derivative (11b) is 2-dimethylaminopyridine.

3. The method for producing the oligonucleic acid derivative according to claim 1, wherein the phenoxyacetic acid derivative anhydride (11a) is phenoxyacetic anhydride.

4. The method for producing the oligonucleic acid derivative according to claim 1, wherein WG¹ is cyano.

5. The method for producing the oligonucleic acid derivative according to any one of claims 1 to 4, **characterized by** using pyridine or 2,6-lutidine.

6. A method for producing an oligonucleic acid represented by the following general formula (A), **characterized by** including the following step, 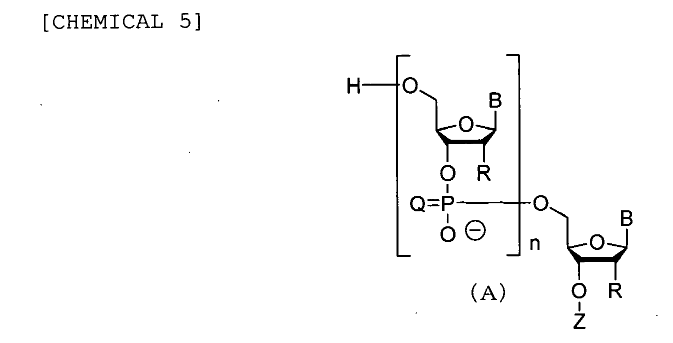 In the general formula (A), each B independently represents a nucleobase or a modified form thereof. n represents an integer in the range of 1 to 200. Each Q independently represents O or S. Each R independently represents H, hydroxyl, halogen, alkoxy, alkylthio, alkylamino, dialkylamino, alkenyloxy, alkenylthio, alkenylamino, dialkenylamino, alkynyloxy, alkynylthio, alkynylamino, dialkynylamino or alkoxyalkyloxy. Z represents H, a phosphate group or a thiophosphate group.
Step:
A process for producing an oligonucleic acid derivative represented by the following general formula (12), **characterized by** using a phenoxyacetic acid derivative anhydride represented by the following general formula (11a) as an acylating agent and a pyridine derivative represented by the following general formula (11b) or (11c) as the acylation reaction activator, in a capping step for protecting the 5'-hydroxyl group of a ribose of an oligonucleic acid derivative represented by the following general formula (2) with an acyl group. 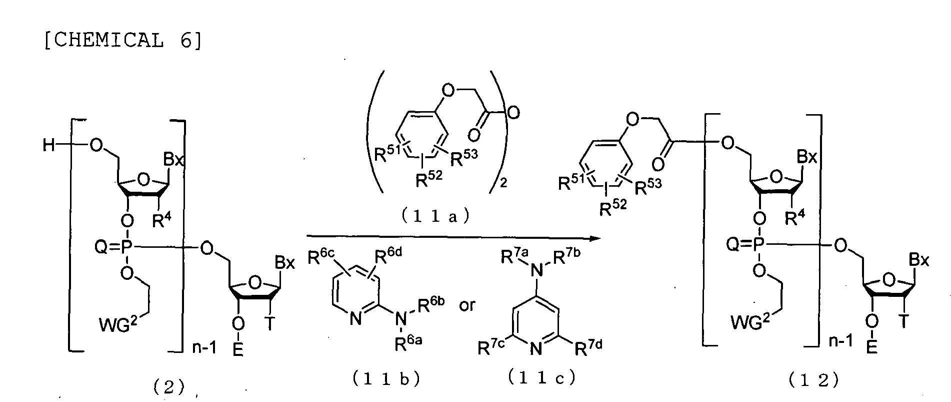
In the general formulae (2), (11a), (11b), (11c) and (12), each Q has the same meanings as above. n has the same meanings as above. Each Bₓ independently represents a nucleobase which may have protecting groups or a modified form thereof. Each WG² represents an electron-withdrawing group. R⁵¹, R⁵² and R⁵³ are the same or different and each represents H, alkyl or halogen. R^{6a}, R^{6b}, R^{7a}, R^{7b}, R^{7c} and R^{7d} are the same or different and each represents alkyl. R^{6c} and R^{6d} are the same or different and each represents H or alkyl. Each R⁴ independently represents H, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy or a substituent represented by the following general formula (3). 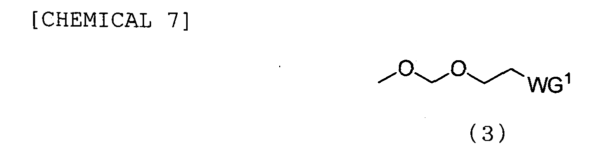
In the general formula (3), WG¹ represents an electron-withdrawing group. E represents acyl or a substituent represented by the following general formula (4). 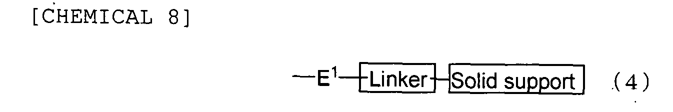
In the general formula (4), E¹ represents a single bond or a substituent represented by the following general formula (5). 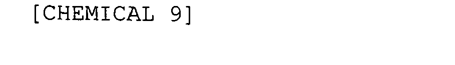 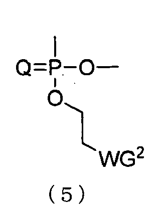
In the general formula (5), Q and WG² have the same meanings as above. T represents H, acyloxy, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy, a substituent represented by the above general formula (3) or a substituent represented by the above general formula (4), with the proviso that either E or T is a substituent (4).

7. The method for producing the oligonucleic acid according to claim 6, wherein a pyridine derivative (11b) is 2-dimethylaminopyridine.

8. The method for producing the oligonucleic acid according to claim 6, wherein the phenoxyacetic acid derivative anhydride (11a) is phenoxyacetic anhydride.

9. The method for producing the oligonucleic acid according to claim 6, wherein WG¹ is cyano.

10. The method for producing the oligonucleotide according to any one of claims 6 to 9, which is **characterized by** further using pyridine or 2,6-lutidine.

11. A method for producing an oligonucleic acid represented by the following general formula (A), **characterized by** including the following Steps A to H, 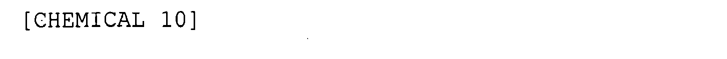 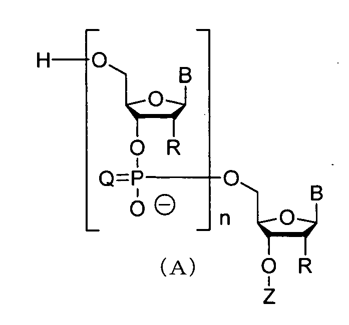 In the general formula (A), each B independently represents a nucleobase or a modified form thereof. n represents an integer between 1 and 200. Each Q independently represents O or S. Each R independently represents H, hydroxyl, halogen, alkoxy, alkylthio, alkylamino, dialkylamino, alkenyloxy, alkenylthio, alkenylamino, dialkenylamino, alkynyloxy, alkynylthio, alkynylamino, dialkynylamino or alkoxyalkyloxy. Z represents H, a phosphate group or a thiophosphate group.
Step A:
Process for producing an oligonucleic acid derivative represented by the following general formula (2) by removing the 5'-hydroxyl group from an oligonucleic acid derivative represented by the following general formula (1) by allowing an acid to act on it. 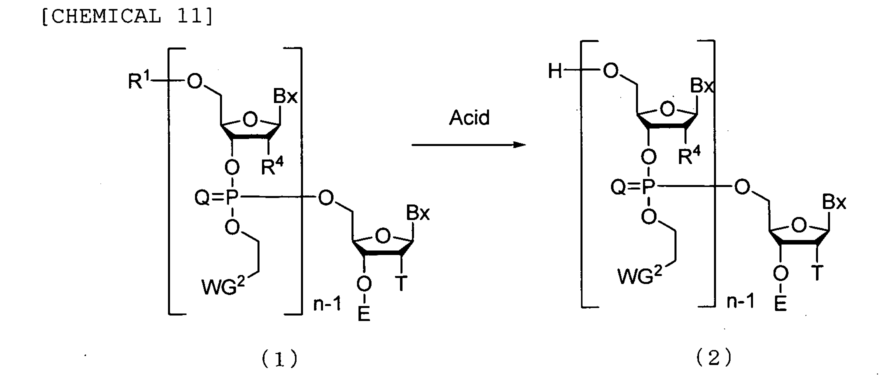
In the general formulae (1) and (2), each Q independently has the same meanings as above. n has the same meanings as above. Each Bₓ independently represents a nucleobase which may have protecting groups or a modified form thereof. R¹ represents a substituent represented by the following general formula (10). 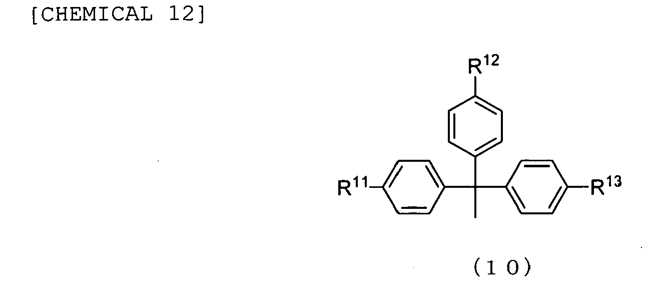
In the general formula (10), R¹¹, R¹² and R¹³ are the same or different and each represents hydrogen or alkoxy. Each WG² represents an electron-withdrawing group. Each R⁴ independently represents H, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy or a substituent represented by the following general formula (3). 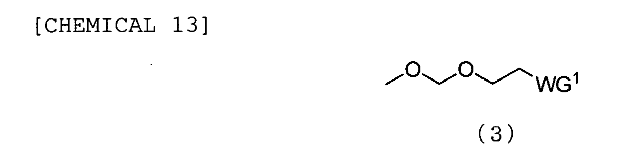
In the general formula (3), WG¹ represents an electron-withdrawing group. E represents acyl or a substituent represented by the following general formula (4). 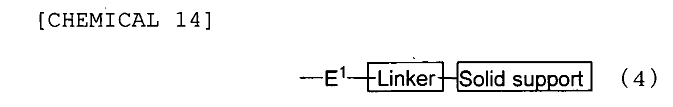
In the general formula (4), E¹ represents a single bond or a substituent represented by the following general formula (5). 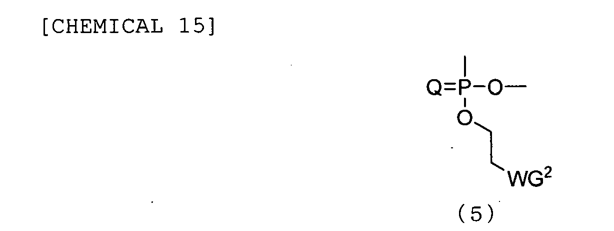
In the general formula (5), Q and WG² have the same meanings as above.
T represents H, acyloxy, halogen, alkoxy, alkylthio, alkylamino, dialkylamino, alkenyloxy, alkenylthio, alkenylamino, dialkenylamino, alkynyloxy, alkynylthio, alkynyl amino, dialkynylamino, alkoxyalkyloxy, a substituent represented by the above general formula (3) or a substituent represented by the above general formula (4), with the proviso that either E or T is a substituent (4).
Step B:
Process for producing an oligonucleic acid derivative represented by the following general formula (9) by condensing a nucleic acid monomer compound with the oligonucleic acid derivative (2) produced in Step A using an activating agent. 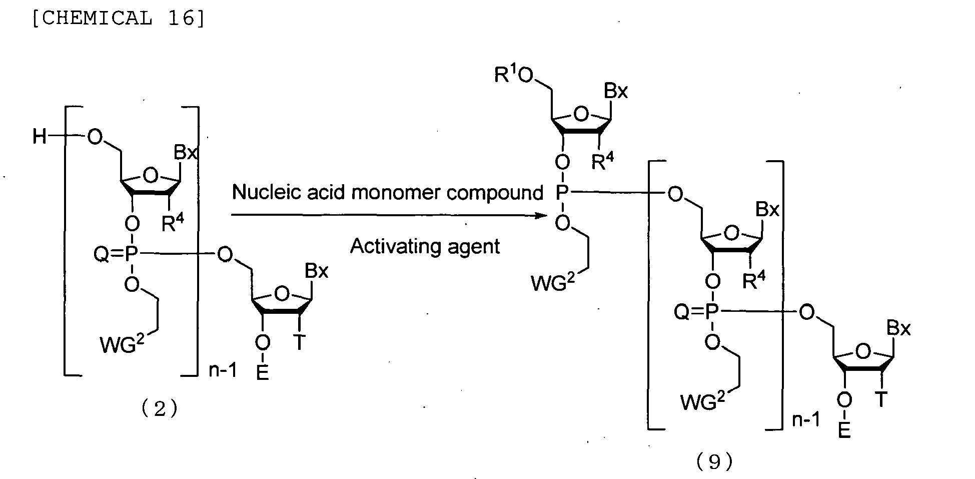
In the general formulae (2) and (9), each B_{X}, each Q, each R⁴ and each WG² independently have the same meanings as above. E, n, R¹ and T have the same meanings as above.
Step C:
Process for producing an oligonucleic acid derivative represented by the following general formula (12), **characterized by** using a phenoxyacetic acid derivative anhydride represented by the following general formula (11a) as an acylating agent and a pyridine derivative represented by the following general formula (11b) or (11c) as the acylation reaction activator, in a capping step for protection with an acyl group of the 5'-hydroxyl group of a ribose of an unreacted oligonucleic acid derivative (2) in Step B. 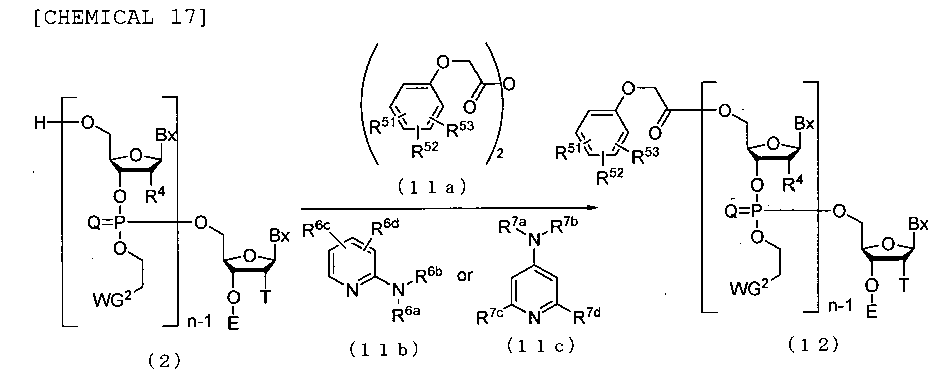
In the general formulae (2), (11a), (11b), (11c) and (12), each Bₓ, each Q, each R⁴ and each WG² independently have the same meanings as above. E, n and T have the same meanings as above. R⁵¹, R⁵² and R⁵³ are the same or different and each represents H, alkyl or halogen. R^{6a}, R^{6b}, R^{7a}, R^{7b}, R^{7c} and R^{7d} are the same or different and each represents alkyl. R^{6c} and R^{6d} are the same or different and each represents H or alkyl.
Step D:
Process for converting a phosphite group into a phosphate group or a thiophosphate group by reacting the oligonucleic acid derivative (9) produced in Step B with an oxidizing agent. 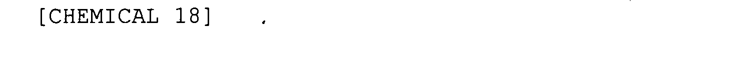 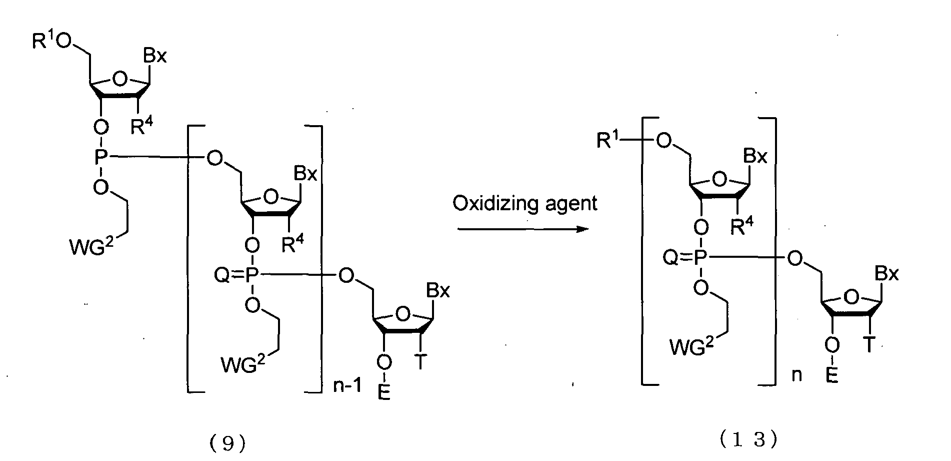
In the general formulae (9) and (13), each B_{X}, each Q, each R⁴ and each WG² independently have the same meanings as above. E, n, R¹ and T have the same meanings as above.
Step E:
Process for cleaving the oligonucleic acid derivative (13) produced in Step D from the solid support, and then deprotecting each nucleobase and each phosphate group, 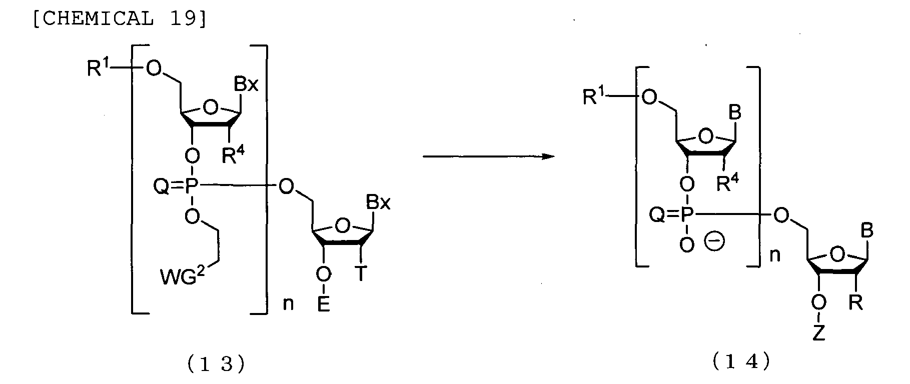
In the general formulae (13) and (14), each B, each Bₓ, each Q, each R⁴ and each WG² independently have the same meanings as above. E, n, R, R¹, T and Z have the same meanings as above
Step F:
Process for producing an oligonucleic acid derivative represented by the following general formula (15) by reacting a reagent for removing the 2'-hydroxyl protecting group of each ribose in the oligonucleic acid derivative (14) produced in Step E, 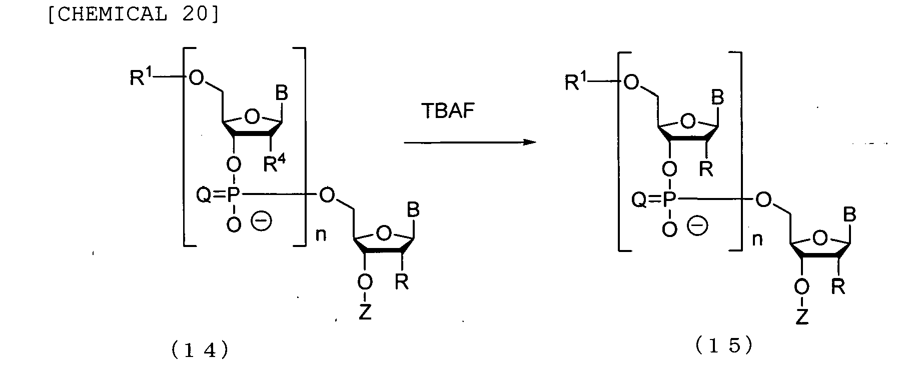
In the general formulae (14) and (15), each B, each Q, each R and each R⁴ independently have the same meanings as above. n, R¹ and Z have the same meanings as above.
Step G:
Process for removing the 5'-hydroxyl group of the oligonucleic acid derivative (15) produced by Step F, 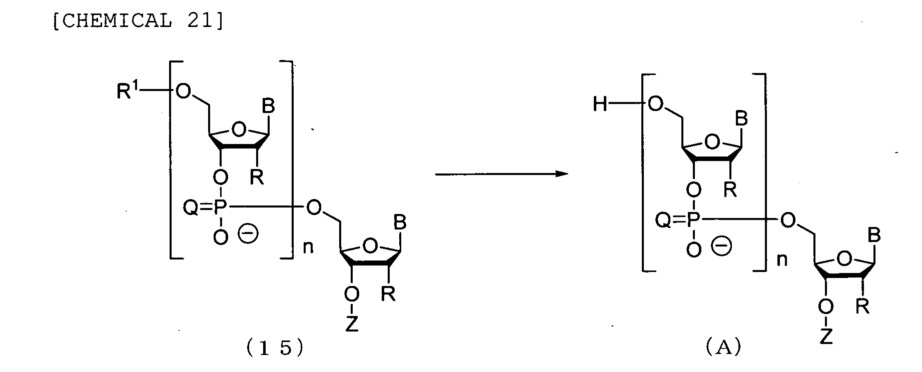
In the general formulae (15) and (A), each B, each Q and each R independently have the same meanings as above. n, R¹ have Z have the same meanings as above.
Step H:
Process for isolating and purifying the oligonucleic acid (A) produced in Step G.

12. The method for producing the oligonucleic acid according to claim 11, which is **characterized by** using one or more a compound represented by the following general formula (B) in Step B for at least one of the nucleic acid monomer compounds. 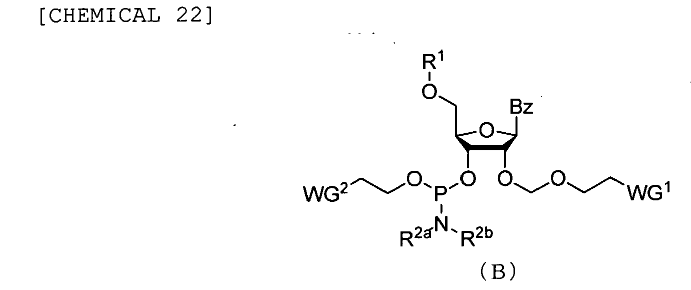 In the general formula (B), B_{Z} represents a nucleobase which may have protecting groups or a modified form thereof. R¹ represents a substituent represented by the following general formula (10). 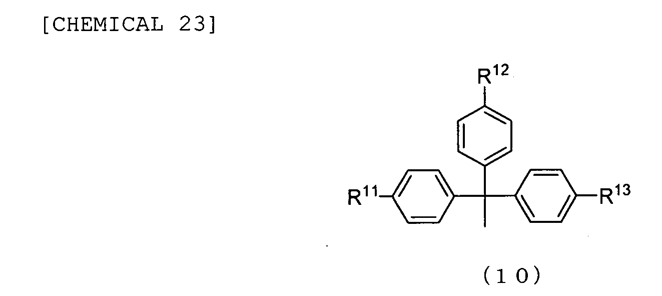 In the general formula (10), R¹¹, R¹² and R¹³ are the same or different and each represents hydrogen or alkoxy. R^{2a} and R^{2b} are the same or different and each represents alkyl, or R^{2a} and R^{2b}, together with the adjacent nitrogen atom, may form a 5- or 6-membered saturated cyclic amino group, the cyclic amino group optionally having one oxygen atom or one sulfur atom as a ring-forming member in addition to the adjacent nitrogen atom. WG¹ and WG² are the same or different and each represents an electron-withdrawing group.

13. The method for producing the oligonucleic acid having a desired chain-length according to claim 11 or 12 by repeating Steps A to D.

14. The method for producing the oligonucleic acid according to any one of claims 11 to 13, wherein pyridine derivative (11b) is 2-dimethylaminopyridine.

15. The method for producing the oligonucleic acid according to any one of claims 11 to 13, wherein the phenoxyacetic acid derivative anhydride (11a) is phenoxyacetic anhydride.

16. The method for producing the oligonucleic acid according to any one of claims 11 to 13, wherein WG¹ is cyano.

17. The method for producing the oligonucleotide according to any one of claims 11 to 16, which is **characterized by** further using pyridine or 2,6-lutidine.
